# EUROPEAN PATENT APPLICATION

(11) **EP 0 977 036 A1**
(43) Date of publication of application: **02.02.2000**
(21) Application number: 97944128.4
(22) Date of filing: 15.10.1997
(51) Int. Cl.: G01N 33/53, C12N 15/31, C12N 1/21, C07K 14/42

(54) **METHOD FOR ASSAYING AGALACTO-IgG AND ASSAY KITS, POLYPEPTIDES OF LECTINS, AND DNAS ENCODING THE SAME**

(30) Priority: 15.10.1996 JP 27273196; 15.10.1996 JP 27273296; 23.07.1997 JP 19762897
(71) Applicant: SEIKAGAKU CORPORATION, Chuo-ku, Tokyo 103-0023 (JP)
(72) Inventor: ICHO, Tateo, Tokyo 182 (JP); MIYAURA, Shuichi, Yokohama-shi Kanagawa 225 (JP); ASANO, Yasushi, Midorimachi Owariasahi-shi Aichi488-0822 (JP); AKIYOSHI, Junko, Tama-shi Tokyo 206 (JP)
(74) Representative: Bannerman, David Gardner
(86) International application number: JP9703723
(87) International publication number: WO9816825

(57) **Abstract**

A method for assaying an agalacto-IgG in a sample, which comprises a step of reacting the agalacto-IgG in the sample with a lectin immobilized on a solid phase to form a complex of the agalacto-IgG and the lectin immobilized on the solid phase; a method for detecting rheumatism by using the assay method; a kit for assaying an agalacto-IgG, and a kit for diagnosing rheumatism, as well as polypeptides of lectins usable in the methods and the kits; and DNAs encoding the same.

## Description

### Technical Field

The present invention relates to a method for assaying an agalacto-IgG utilizing a lectin immobilized on a solid phase, and a kit for assaying an agalacto-IgG. The present invention also relates to a method for detecting rheumatism characterized in that an agalacto-IgG is measured by the method for assaying the agalacto-IgG utilizing the lectin immobilized on the solid phase, a kit for assaying an agalacto-IgG, and a kit for diagnosing rheumatism. The present invention further relates to a polypeptide having the same activity as that of *Psathyrella velutina* lectin (PVL), and a DNA encoding *Psathyrella velutina* lectin or its equivalent having the same activity.

### Background Art

Rheumatoid arthritis is a disease which exhibits polyarthritis as a predominant symptom, and is accompanied by broad inflammation in organ tissues of the whole body. It has been known that an autoantibody recognizing an epitope which exists in the Fc region of immunoglobulin G (IgG), which is called rheumatoid factor, is present in sera of rheumatoid arthritis patients. This suggests that IgG of rheumatism patients producing the rheumatoid factors has a structure different from IgG of normal subjects. Detailed analysis of sugar chain structures of the Fc region of IgG in rheumatism patient sera has revealed that IgG having a sugar chain lacking a galactose (Gal) residue (agalacto-IgG) is markedly increased in rheumatism patient sera. That is, it has been revealed that the sugar chain moiety in the Fc region of IgG in normal subject sera fundamentally has a complex type binary sugar chain structure comprising a mixture of 16 kinds of oligosaccharides, which are arisen from presence or absence of a Gal residue, presence or absence of a fucose residue, and presence or absence of an N-acetylglucosamine (GlcNAc) residue bound to a β-mannose residue, and relative ratios of these 16 kinds of oligosaccharides are constant in IgG of normal subjects, whereas the sugar chains present in the Fc region of IgG in rheumatism patient sera have an increased ratio of sugar chains lacking both of the two Gal residues though it also has a complex type binary structure. Accordingly, structural abnormality occurs in the sugar chain moiety in the Fc region of IgG in rheumatism patient sera, and this abnormality of sugar chain structure would play a role as a marker of the rheumatoid factor.

The current methods for diagnosing rheumatoid arthritis are mainly those measuring the rheumatoid factor (autoantibody to agalacto-IgG), and they measure the rheumatoid factor which reacts with the modified IgG by utilizing latex agglutination reaction. The rheumatoid factor can be classified into three classes, i.e. those of IgG, IgM and IgA, and sensitivity and specificity of diagnostic methods have recently been argued through measurement of each rheumatoid factor. As described above, it is considered that the causative agent for these rheumatoid factors is the agalacto-IgG resulting from the abnormality of sugar chain of IgG. Therefore, it is considered important for the diagnosis of rheumatoid arthritis to directly assay the agalacto-IgG which is the causative agent.

As a method for assaying the agalacto-IgG, the technique disclosed in Japanese Patent Application Laid-Open No. 5-87814 (1993) can be mentioned. The fundamental principle of this technique is measurement of the amount of normal IgG by sandwiching the normal IgG in human serum between an anti-human IgG antibody and a lectin (RCA; *Ricinus communis* agglutinin) which is reactive with normal human IgG (this is merely mentioned as "human IgG" in the above patent document). In other words, this is an assay method for estimating the increased amount of the agalacto-IgG by measuring the decreased amount of normal IgG based on an idea that the amount of normal IgG in blood of rheumatoid arthritis patients decreases, because the normal IgG is abnormally metabolized into the agalacto-IgG. However, this assay method still has a problem that it is difficult to obtain an accurate amount of the agalacto-IgG by this method, because the estimated agalacto-IgG amount may fluctuate due to the variation of the total IgG amount in blood, and it is not a direct quantitation method of the agalacto-IgG. While the aforementioned patent document discloses use of an anti-human IgG antibody immobilized on a solid phase (insoluble carrier), it does not disclose use of a lectin immobilized on a solid phase as in the present invention. Further, the aforementioned patent document discloses a technique for determining the amount of the agalacto-IgG based on the amount of normal IgG measured by binding IgG in human serum (mixture of the normal IgG and the agalacto-IgG) to the anti-human IgG antibody immobilized on the solid phase, and recognizing only the normal IgG among the IgG with a lectin (RCA) (i.e., sandwiching the normal IgG between the anti-human IgG antibody and the lectin (RCA)), but it does not disclose any technique for directly sandwiching the agalacto-IgG between an anti-human IgG antibody and a lectin (direct quantitative assay method for the agalacto-IgG). The present invention has a constitution distinctly different from those of the invention disclosed in the aforementioned patent document, and provides remarkable advantages that cannot be obtained by the invention disclosed in the aforementioned patent document. These advantages will be explained hereinafter.

A direct quantitative assay method for the agalacto-IgG has been reported by Tsuchiya et al. (J. Immunol., 151, 1137-1146 (1993)). In this method, the agalacto-IgG is sandwiched between protein G and *Psathyrella velutina* lectin (PVL) labeled with biotin. Therefore, this method requires expensive protein G and PVL labeled with biotin. In particular, it is not easy to label PVL with biotin because PVL has an agglutination property, and this method also suffer a serious problem concerning stability of PVL labeled with biotin. The present invention also has a constitution distinctly different from those of the invention described in the above literature, and provides remarkable advantages that cannot be obtained by the invention disclosed in the above literature. These advantages will also be explained hereinafter.

Considering these conventional techniques, a method for easily carrying out direct quantitative assay of the agalacto-IgG with high sensitivity and at low cost is desired. That is, an object of the present invention is to provide a method for easily carrying out direct quantitative assay of the agalacto-IgG in a sample with high sensitivity and at low cost. Another object of the present invention is to provide a method for detecting rheumatism, in particular, rheumatoid arthritis, by using the above assay system. A further object of the present invention is to provide a kit for assaying the agalacto-IgG, and a kit for diagnosing rheumatism.

The properties and the production method of the *Psathyrella velutina* lectin (abbreviated as "PVL" hereinafter) are disclosed in Japanese Patent Application Laid-Open No. 1-139599 (1999). As for the purification method and properties of PVL, the article of Kochibe has been published (J. Biol. Chem., 264, 173-177 (1989)). Further, as for the binding characteristics of PVL to the IgG sugar chain lacking galactose, the article of Tsuchiya (supra) has been published. However, a DNA encoding PVL and an amino acid sequence of PVL have not been known yet.

If a DNA encoding PVL is provided, its use for production of the lectin in a recombinant expression system and modification of the properties of the lectin can be expected. Therefore, further objects to be achieved by the present invention are to provide a DNA encoding PVL, and to provide a novel polypeptide of lectin based on the DNA.

### Disclosure of the Invention

The present inventors have found that the problems concerning the agglutination property of lectin and the unstability of labeled lectin can be solved and a highly sensitive assay of the agalacto-IgG can be provided by immobilizing the lectin reactive to the agalacto-IgG on a solid phase, and succeeded in providing an inexpensive and simple assay method for the agalacto-IgG and the like. Thus, the present invention has been accomplished.

The present invention relates to a method for assaying an agalacto-IgG in a sample by reacting the agalacto-IgG in the sample with a lectin immobilized on a solid phase to form a complex of the agalacto-IgG and the lectin immobilized on the solid phase, a method for detecting rheumatism, in particular, rheumatoid arthritis by using the aforementioned assay method, a kit for assaying an agalacto-IgG, and a kit for diagnosing rheumatism.

The formed complex is preferably detected with an anti-IgG antibody. More preferably, the method comprises the step of reacting the agalacto-IgG in the sample with the lectin immobilized on the solid phase to form the complex of the agalacto-IgG and the lectin immobilized on the solid phase, and a step of reacting the agalacto-IgG with the anti-IgG antibody. More preferably, the step of reacting the agalacto-IgG with the anti-IgG antibody is performed after the step of forming the complex. The lectin is preferably a lectin which specifically binds to a β-N-acetylglucosamine residue, particularly preferably a *Psathyrella velutina* lectin (PVL).

The present invention also relates to a method for assaying an agalacto-IgG in a sample which comprises a step of sandwiching the agalacto-IgG between a lectin and an anti-IgG antibody. The lectin is preferably immobilized on a solid phase. The lectin is preferably a lectin which specifically binds to a β-N-acetylglucosamine residue, particularly preferably a *Psathyrella velutina* lectin (PVL).

As a diagnostic method of rheumatoid arthritis, there have been reported the method for assaying rheumatoid factor, the method for indirectly assaying the agalacto-IgG which is the causative agent, and the method for directly assaying the agalacto-IgG, which requires highly developed techniques and expensive reagents. However, according to the present invention, there is provided a simple and inexpensive method for directly assaying the agalacto-IgG, and simple, inexpensive and highly sensitive detection of rheumatoid arthritis can be realized by the assay method. Further, a kit therefor is also provided.

Further, the present inventors have successfully cloned cDNA encoding PVL, and finally succeeded in providing a polypeptide of lectin having a specific amino acid sequence and partial polypeptides thereof (also collectively referred to as "polypeptide of the present invention" hereinafter), antibodies which bind thereto, and DNAs encoding a polypeptide of lectin such as PVL and partial polypeptides thereof (also referred to as "DNA of the present invention" hereinafter).

The polypeptide of the present invention include a polypeptide of lectin which comprises at least an amino acid sequence represented by the following formula (1):
Asp Xaa Xaa Gly Phe Gly Xaa Xaa Xaa Xaa Xaa Xaa Xaa Xaa Asn (Xaa) (Gly) (Xaa) Xaa Xaa Xaa (Xaa) Xaa Xaa Xaa Xaa (Xaa) Xaa Xaa Xaa Xaa Xaa Xaa Xaa (Xaa) Xaa Xaa Gly Trp Xaa Xaa Xaa Xaa Xaa Xaa Arg [Formula (1)]
wherein each Xaa is an arbitrary amino acid, and each parenthesized amino acid may be absent.

The polypeptide of the present invention include a polypeptide of lectin which comprises at least a part of the amino acid sequence shown in SEQ ID NO: 2, and may have substitution, deletion or insertion of one or more amino acid residues that does not substantially spoil its binding activity to a sugar chain (preferably a sugar chain having an N-acetylglucosamine residue, more preferably a sugar chain having an N-acetylglucosamine residue at its end, particularly preferably an N-acetylglucosamine residue of a sugar chain having the N-acetylglucosamine residue at its end, extremely preferably an N-acetylglucosamine residue of a sugar chain having the N-acetylglucosamine residue binding a mannose residue (GlcNAc → Man) at its end). Preferably, the polypeptide of the present invention is a polypeptide having at least a part of the amino acid sequence shown in SEQ ID NO:2.

The polypeptide of the present invention further include a polypeptide comprising a portion of the polypeptides mentioned above.

There are further provided a fusion polypeptide comprising any of the aforementioned polypeptides and another polypeptide in a polypeptide chain, and an antibody which binds to the polypeptide of the present invention.

The DNA of the present invention includes a DNA encoding a lectin having the following properties:
(1) binding to a sugar chain (preferably a sugar chain having an N-acetylglucosamine residue, more preferably a sugar chain having an N-acetylglucosamine residue at its end, particularly preferably an N-acetylglucosamine residue of a sugar chain having the N-acetylglucosamine residue at its end, extremely preferably an N-acetylglucosamine residue of a sugar chain having GlcNAc → Man containing the N-acetylglucosamine residue at its end),
(2) having a molecular weight of about 40 kilodalton as estimated by sodium dodecyl sulfate (SDS)-polyacrylamide gel electrophoresis (SDS-PAGE),
(3) having the following amino acid composition:

| Residue | Mol % |
|---|---|
| Asx | 14.9 |
| Thr | 5.5 |
| Ser | 3.2 |
| Glx | 4.2 |
| Pro | 3.2 |
| Gly | 14.9 |
| Ala | 8.0 |
| Cys | 1.0 |
| Val | 10.4 |
| Met | 1.5 |
| Ile | 5.2 |
| Leu | 7.0 |
| Tyr | 2.5 |
| Phe | 5.5 |
| His | 1.7 |
| Lys | 3.7 |
| Trp | 2.2 |
| Arg | 5.2 |

(4) present in fruiting body of *Psathyrella velutina*.

The DNA of the present invention also include a DNA encoding at least a part of the above polypeptide of the present invention.

The DNA of the present invention preferably contains a nucleotide sequence encoding the amino acid sequence represented by the amino acid numbers 1-402 in SEQ ID NO:2, more preferably a part or all of the nucleotide sequence represented by the nucleotide numbers 41-1246 in SEQ ID NO:1.

Further, there are provided a recombinant DNA and a recombinant vector each comprising the DNA of the present invention, and a transformant cell obtainable by introducing the vector into a host cell.

The lectin comprising the above polypeptide of the present invention can be used as the lectin in the aforementioned method for assaying the agalacto-IgG.

### Brief Description of the Drawings

Figure 1 (A) is a graph representing results of analysis of a prepared anti-PVL antibody for reactivity with PVL by ELISA, and Figure 1 (B) is a schematic view representing results of analysis of a prepared anti-PVL antibody for reaction specificity by Western blotting.
Figure 2 shows a fundamental strategy (sequencing strategy) for determining the nucleotide sequence of PVL cDNA clone, SKPVL231.
Figure 3 shows a nucleotide sequence and an amino acid sequence of a PVL cDNA clone, and their relationship with PVL partial amino acid sequences.
Figure 4 shows repeats in a PVL amino acid sequence.
Figure 5 shows a schematic structure of a PVL expression plasmid (pPPVL231).
Figure 6 shows an example of expression of recombinant PVL in *E. coli* (photographs of electrophoresis gel).
Figure 7 shows a schematic structure of a PVL expression plasmid (pPICPVL231).
Figure 8 shows a calibration curve of agalacto-IgG in Comparative Example 1.
Figure 9 shows a calibration curve of agalacto-IgG of Example 3.
Figure 10 shows influence of various monosaccharides on an assay system determined in Example 4.
Figure 11 shows agalacto-IgG concentrations in sera of normal subjects, OA patients, RA patients and liver disease patients determined in Example 5.

### Best Mode for Carrying Out the Invention

The present invention will be explained in detail hereinafter.

### 〈1〉 Assay method for agalacto-IgG

The assay method of the present invention comprises at least a step of forming a complex of a lectin immobilized on a solid phase and an agalacto-IgG in a sample.

As the solid phase to which the lectin is immobilized used for the assay method of the present invention, there can be mentioned plates, tubes, beads, membranes, gel and the like. As materials of the solid phase, polystyrene, polypropylene, nylon, latex, glass, crosslinked dextrin, agarose, crosslinked agarose, polyacrylamide and the like can be mentioned.

As the method for immobilizing the lectin to the solid phase, usual methods for preparing immobilized enzymes such as physical adsorption, covalent bond method, and entrapment method (See, "Immobilized Enzyme", 1975, published by Kodansha, pp.9-75) can be utilized. The physical adsorption is particularly preferred because of ease of operation.

On the surface of the solid phase to which the lectin has been immobilized, an area where the lectin is not immobilized may remain. If the agalacto-IgG in the sample or other molecular species adheres to the area, accurate assay results may not be obtained. Therefore, it is preferable to coat the area where the lectin is not immobilized, by adding a blocking agent before the sample is brought into contact with the solid phase. Examples of the blocking agent include serum albumin obtained from mammals such as bovine, casein, milk protein, lactic acid fermentation products, collagen, degraded products thereof and the like, and those products commercially available as blocking agents may also be used.

It is preferred that the surface of the solid phase is washed with a washing solution to remove non-specifically adsorbed substances after the agalacto-IgG in the sample has been bound to the lectin immobilized on the solid phase. Examples of the washing solution include buffers (e.g., phosphate buffer, phosphate buffered saline (PBS), Tris-HCl buffer etc.) to which detergents such as Tween series detergents are added.

IgG is a glycoprotein which constitutes about 80% of the immunoglobulin in blood, is present at a concentration of about 10 to 15 mg/ml, and has a half life of 16 to 23 days. A complex type binary sugar chain is bound to the asparagine at position 297 of the CH2 region, and it contains one or two molecules of Gal residue in normal subjects. On the other hand, Gal is markedly decreased in the sugar chain of IgG molecules in blood of rheumatoid arthritis patients, and GalNAC molecules are exposed at the both ends of the complex type binary sugar chain. It is considered that this sugar chain abnormality becomes to exhibit antigenicity, and thus an autoantibody (rheumatoid factor) is produced. The antibody having this sugar chain abnormality (lack of Gal) is agalacto-IgG.

While the lectin is not particularly limited so long as it reacts with the agalacto-IgG, it is preferably a lectin which is specifically bound to a β-N-acetylglucosamine (β -GlcNAc) residue. Examples of the lectin include a lectin derived from *Triticum vulgaris*, a lectin derived from *Datura stramonium*, a lectin derived from *Lycopersicon esculentum*, a lectin derived from *Soianum tuberosum*, a lectin derived from *Laburnum alpinum*, a lectin derived from *Oryza sativa*, a lectin derived from *Phytolacca american*a, a lectin derived from *Ulex europaeus* II, a lectin derived from *Psathyrella velutina* (*Psathyrella velutina* lectin, PVL) and the like.

Among these lectins, the *Psathyrella velutina* lectin (PVL) is particularly preferred, because it has surely been confirmed to react with the agalacto-IgG, and not react with normal IgG. The lectin comprising the polypeptide of the present invention, which will be explained in the section 〈5〉 hereinafter, can also be used.

PVL is a protein which is present in fruiting body of *Psathyrella velutina*, and has a molecular weight of about 40000. This lectin exhibits higher affinity for GlcNAc compared with conventional lectins, and in particular, it exhibits higher affinity to GlcNAc β1→6 linkage and GlcNAc β1→3 linkage compared with GlcNAc β1→4 linkage. It has also been reported that this PVL reacts with GalNAc of the complex type binary sugar chain present in the agalacto-IgG molecule.

PVL used for the present invention may either be commercially available PVL, PVL extracted and purified from fruiting body of *Psathyrella velutina* lectin by the known method (Japanese Patent Application Laid-Open No. 1-139599 (1989)), or a recombinant PVL obtained by a known genetic engineering technique.

In the assay method of the present invention, the method for detecting the formed complex of the agalacto-IgG and the lectin is not particularly limited. Because an anti-IgG antibody reacts with the agalacto-IgG, the detection of the complex of the agalacto-IgG and the lectin is preferably performed by using the anti-IgG antibody. The detection of the complex of the agalacto-IgG and the lectin may also be performed either by, after the formation of the complex, reacting the formed complex with the anti-IgG antibody, or reacting the agalacto-IgG and the anti-IgG antibody first, and then forming the complex of the agalacto-IgG and the lectin. Preferably, the detection is performed by forming the complex of the agalacto-IgG and the lectin, and then reacting the complex with the anti-IgG antibody.

The anti-IgG antibody is preferably an anti-IgG antibody labeled with a labeling substance, or an anti-IgG antibody which can be labeled with a labeling substance. It is preferably an anti-human IgG antibody.

The anti-human IgG antibody which can be used for the present invention may be commercially available anti-human IgG antibodies, or monoclonal antibodies or polyclonal antibodies which are obtained by immunizing an animal such as mouse, rat, guinea pig, hamster, rabbit, goat, and sheep with commercially available human IgG. The aforementioned commercially available anti-human IgG antibodies are preferred because they are obtained easily. The recognition and binding sites of both of protein G and PVL congest in the Fc region of an agalacto-IgG molecule, and the anti-human IgG antibody also recognizes a site other than the Fc. Therefore, the anti-human IgG antibody is preferred for the formation of the sandwich complex with the agalacto-IgG.

The present invention include a technique for directly detecting the agalacto-IgG by sandwiching the agalacto-IgG with the lectin and the anti-IgG antibody, namely, a method for assaying the agalacto-IgG in the sample comprising a step of sandwiching the agalacto-IgG between the lectin and the anti-IgG antibody. The lectin is preferably immobilized on a solid phase, and preferred lectins, preferred anti-IgG antibodies, and other preferred embodiments are similar to those described above.

The present invention will be exemplified for the case utilizing PVL and an anti-human IgG antibody hereinafter. Other lectins and other anti-IgG antibodies can be used similarly.

To measure the agalacto-IgG in the sample using PVL in the present invention, the so-called sandwich method (see Japanese Patent Publication No. 6-41952 (1994) etc.) is usually used. That is, the method comprises forming a sandwich complex composed of three kinds of components, i.e., the agalacto-IgG, PVL and the anti-human IgG antibody, utilizing affinity between a sugar chain in the agalacto-IgG molecule and PVL, and affinity of the anti-human IgG antibody for the protein portion of the agalacto-IgG molecule, and measuring the complex. In this method, PVL is usually immobilized on a solid phase to separate the sandwich complex for measurement. For example, in this method, a sample containing the agalacto-IgG is brought into contact with PVL immobilized on a solid phase, then with an anti-human IgG antibody labeled with a labeling substance to cause reaction of the agalacto-IgG in the sample, PVL and the labeled anti-human IgG antibody, thereby forming a complex of solid phase/PVL/agalacto-IgG/labeled anti-human IgG antibody, the complex and unbound labeled anti-human IgG antibodies are separated, and the labeling substance in the complex is measured to assay the agalacto-IgG in the sample.

It is also possible to assay the agalacto-IgG in the sample by contacting a complex of solid phase/PVL/agalacto-IgG/anti-human IgG antibody formed by using an unlabeled anti-human IgG antibody, with a labeled anti-(anti-human IgG) antibody which is specifically bound to the anti-human IgG antibody to form a complex of the complex and the labeled anti-(anti-human IgG) antibody, and detecting the labeling substance of the complex. The anti-(anti-human IgG) antibody which can be used in this method is an antibody which recognizes immunoglobulins of animals allogeneic with the animal immunized with human IgG in order to produce the anti-human IgG antibody.

In the aforementioned sandwich method, the order of the PVL/agalacto-IgG/anti-human IgG antibody complex formation may be either the so-called forward, or reverse, or simultaneous mode (see "Protein, Nucleic acid, Enzyme", Separate volume No. 31, Enzyme Immunoassay, Kyoritsu Shuppan Co., Ltd., 1987, pp.13-26).

Examples of the labeling substance used for labeling the anti-human IgG antibodies or anti-immunoglobulin antibodies having immunological affinity for the anti-human IgG antibodies (anti-(anti-human IgG) antibody) include enzymes such as peroxidase, alkaline phosphatase, β-galactosidase, luciferase, and acetylcholinesterase; isotopes such as ¹²⁵I, ¹³¹I and ³H; fluorescent dyes such as luminol, fluorescein isothiocyanate (FITC), umbelliferone, and 7-amino-4-methylcoumarin-3-acetate; chemiluminescent substances; haptens; biotin; avidins such as streptavidin and the like. However, the labeling substance is not particularly limited so long as it can normally be used for labeling of proteins. The labeling substance also include a substance used for a method in which the substance itself (e.g., biotin) is not detected, but a combination of the substance and another substance (e.g., avidin) capable of specifically binding to the former substance and labeled with a detectable marker is utilized.

The method for labeling the antibodies can optionally be selected from known methods suitable for the labeling substances, for example, the glutaraldehyde method, the periodic acid crosslinking method, the maleimide crosslinking method, the carbodiimide method, the activated ester method and the like for labeling with enzymes, the chloramine-T method, the lactoperoxidase method and the like for labeling with radioisotopes (see, "The Second Series of Lecture of Biochemical Experiments 2", Chemistry of Proteins, volume 2, Tokyo Kagaku Dojin, 1987), and the like.

While the detection method of the labeling substance may be vary depending on the labeling substance to be used, examples thereof include, for instance, when biotin is used as the labeling substance, the method which comprises adding an enzyme binding streptavidin or the like so that the enzyme such as peroxidase as a labeling substance should be bound to the complex containing biotin via the streptavidin, adding a chromogenic substrate such as tetramethylbenzidine and hydrogen peroxide as substrates of the enzyme, and measuring degree of color development of the product produced by the enzyme reaction based on the alteration of absorbance. Further, when a fluorescent substance or a chemiluminescent substance is used as the labeling substance, for example, a method comprising measuring fluorescence or luminescence of the solution after the reaction can be used.

In the assay method of the present invention, the concentration of the agalacto-IgG in the sample can be determined by preparing a calibration curve for the relationship between the agalacto-IgG concentration and results of the labeling substance detection using agalacto-IgG standard solutions having known concentrations beforehand, and comparing the detection result for a sample of unknown concentration with the calibration curve.

While the sample for the assay method of the present invention is not particularly limited, it is preferably a liquid sample, more preferably a liquid sample such as synovial fluid, blood, serum, plasma, cell culture medium and the like. In particular, when the assay method is used for the detection of rheumatism, which will be explained hereinafter, body fluid such as synovial fluid, blood, serum, and plasma is preferred as the sample. The body fluid is preferably one derived from human. The sample containing the agalacto-IgG used for the assay method of the present invention needs not to be purified beforehand. That is, the agalacto-IgG can be selectively measured even if concomitants such as other serum proteins are present, and hence the assay results are not affected by them.

A preferred mode of the assay method of the present invention will be explained hereinafter. First, PVL is immobilized on (coated on) a solid phase. The method for the immobilization may comprise, for example, dissolving PVL in a buffer having a pH of about 7 to 9 (for example, phosphate buffer, PBS, carbonate buffer etc.) containing 10% glycerol, adding the solution to a solid phase (e.g., well of microplate), and leaving it stand at a temperature around 37°C for 1 to 2 hours, or at a temperature around 4°C overnight, so that the PVL should be immobilized.

After the immobilization, it is preferable to coat portions on which PVL is not immobilized, by adding a blocking agent such as serum albumin, and leaving the solid phase stand at a temperature around 37°C for 30 minutes to 2 hours, or at ambient temperature (15 to 25°C) for one to 2 hours. Then, a sample containing an agalacto-IgG is added to the aforementioned PVL-immobilized solid phase, and left stand or stirred for a suitable period of time, for example, at 37°C for 20 to 80 minutes so that the agalacto-IgG should be bound to the PVL.

Subsequently, the solid phase to which the complex is bound, is washed with a washing solution, for example, one composed of a buffer such as phosphate buffer, PBS and Tris-HCl buffer to which a Tween series surfactant is added. Further, an anti-human IgG antibody labeled with a labeling substance, or an anti-human IgG antibody and an anti-(anti-human IgG) antibody labeled with a labeling substance are added to the solid phase, and left stand or stirred, for example, at 37°C for 20 to 80 minutes so that the anti-human IgG antibody (or the anti-human IgG antibody and the anti-(anti-human IgG) antibody) should be bound to the agalacto-IgG. By this procedure, a complex of solid phase/PVL/agalacto-IgG/anti-human IgG antibody (or solid phase/PVL/agalacto-IgG/anti-human IgG antibody/anti-(anti-human IgG) antibody) is formed. Then, the labeling substance of the complex is detected to assay the agalacto-IgG.

Separately, a calibration curve is prepared for the relationship between concentration of agalacto-IgG standard solutions and result of labeling substance detection (e.g., absorbance), and a detection result for an unknown sample is compared with the calibration curve to quantitate the agalacto-IgG in the unknown sample.

### 〈2〉 Method for detecting rheumatism

The amount of the agalacto-IgG increases in rheumatism, in particular, rheumatoid arthritis patients as described above. Accordingly, rheumatism can be detected by measuring the agalacto-IgG using the assay method for the agalacto-IgG described above. This detection includes not only determination of presence or absence of rheumatism, but also evaluation of degree of rheumatism.

The amount of the agalacto-IgG which is to be a criterion for detection of rheumatism may be appropriately defined depending on the kind of the sample, and detection can be performed based on the measured agalacto-IgG amount.

The amount of the agalacto-IgG which is to be the criterion for detection of rheumatism may be either an amount obtained by using a calibration curve prepared for the relationship between concentration of agalacto-IgG standards and results of labeling substance detection, or represented as a ratio relative to the agalacto-IgG amount in a sample of normal subject (human not having rheumatoid arthritis) without using the calibration curve.

### 〈3〉 Kit for assaying agalacto-IgG

The present invention also relates to a kit used for a method wherein an agalacto-IgG is assayed by forming a complex of the agalacto-IgG and a lectin, which comprises the lectin immobilized on a solid phase as a component. Preferably, the kit for assaying the agalacto-IgG of the present invention further comprises an anti-IgG antibody, and the anti-IgG antibody is labeled or can be labeled with a labeling substance. More preferably, it essentially comprises a solid phase to which a lectin which is specifically bound to β-N-acetylglucosamine residue (particularly preferably PVL) is immobilized as the lectin, and an anti-human IgG antibody labeled with a labeling substance.

By using a solid phase to which PVL is immobilized beforehand, the process for immobilizing the lectin in the assay method described above can be omitted, and the undesired agglutination of the lectin can also be avoided.

The kit may further comprise agalacto-IgG standards having known concentrations for the preparation of calibration curve, a reagent for detecting a labeling substance, a reagent for labeling the anti-human IgG antibody or a reagent for detecting the anti-human IgG antibody (e.g., a labeled anti-(anti-human IgG) antibody) and the like. In addition to these components, the kit may further comprise the blocking agent, the washing solution, a diluent for sample, an enzyme reaction-terminating solution or the like.

These components may be stored as a kit comprising them in separate containers, which can be used according to the prescription described above.

To agalacto-IgG in a sample such as synovial fluid, blood, serum, plasma, cell culture medium or the like can be specifically measured by using the kit for assaying the agalacto-IgG of the present invention, and it has a broad assay range, and a high sensitivity.

The kit for assaying the agalacto-IgG, comprising PVL immobilized on a solid phase and a labeled anti-human IgG antibody is used, for example, as follows. That is, a sample containing an agalacto-IgG is brought into contact with PVL immobilized on the solid phase, and then with the labeled anti-human IgG antibody to form a complex of solid phase/PVL/agalacto-IgG/labeled anti-human IgG antibody, and the labeling substance contained in the complex is measured to assay the agalacto-IgG concentration in the sample.

### 〈4〉 Kit for diagnosing rheumatism of the present invention

When the agalacto-IgG in human serum is measured by using the kit for assaying the agalacto-IgG of the present invention, extremely higher values are obtained for rheumatoid arthritis patients compared with normal subjects, knee osteoarthritis patients, and liver disease patients. Accordingly, the kit for assaying the agalacto-IgG of the present invention, which can assay the agalacto-IgG with simple procedure and at low cost, can be used as an extremely useful diagnostic kit, i.e., as a diagnostic agent, with which rheumatoid arthritis can be specifically and easily diagnosed.

### 〈5〉 Polypeptide of the present invention

The polypeptide of the present invention include a polypeptide of lectin comprising at least an amino acid sequence represented by the above formula (1).

The polypeptide of the present invention preferably comprises at least a sequence corresponding to one cycle starting from an arbitrary position of a cyclic amino acid sequence represented by the following formula (2):
Xaa Xaa Xaa Asp Xaa Xaa Gly Phe Gly Xaa Xaa Xaa Xaa Xaa Xaa Xaa Xaa Asn (Xaa) (Gly) (Xaa) Xaa Xaa Xaa (Xaa) Xaa Xaa Xaa Xaa (Xaa) Xaa Xaa Xaa Xaa Xaa Xaa Xaa (Xaa) Xaa Xaa Gly Trp Xaa Xaa Xaa Xaa Xaa Xaa Arg Xaa Xaa Xaa Xaa Xaa Xaa Xaa (Xaa) [Formula (2)]
wherein the sequence of the above formula is a cyclic sequence, and wherein each Xaa is an arbitrary amino acid, and each parenthesized amino acid may be absent.

While the "amino acid" used herein generally means one of the 20 kinds of amino acids constituting proteins (alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine), it may include other amino acids. The amino acids other than glycine are preferably L-amino acids.

The "cyclic sequence" used herein means a sequence composed of the sequences of the above Formula (2) repetitively linked in succession, like a sequence of, for example, the first amino acid (Xaa) of the Formula (2) ... the last amino acid ((Xaa))-the first amino acid ... the last amino acid-the first amino acid ... .

The "sequence corresponding to one cycle starting from an arbitrary position of a cyclic amino acid sequence" means, for example, when it is exemplified for the case where Phe at position 8 from the first amino acid in the above Formula (2) is considered the starting position, a sequence of "Phe-Gly-Xaa-Xaa- ... -Asp-Xaa-Xaa-Gly". However, this is mentioned only for illustration, and it is not limited to this sequence.

The polypeptide of the present invention alternatively comprises at least an amino acid sequence represented by the following formula (3):
Asp Xaa1 Xaa1 Gly Phe Gly Xaa Xaa Xaa Xaa1 Xaa Xaa Xaa Xaa Asn (Xaa) (Gly) (Xaa) Xaa Xaa Xaa1 (Xaa) Xaa Xaa1 Xaa1 Xaa (Xaa) Xaa1 Xaa1 Xaa Xaa Xaa1 Xaa Xaa (Xaa) Xaa Xaa Gly Trp Xaa Xaa1 Xaa Xaa Xaa Xaa1 Arg Xaa Xaa1 Xaa1 [Formula (3)]
wherein each Xaa is an arbitrary amino acid, each Xaa1 is a hydrophobic amino acid (in the present specification, it means "Gly, Ala, Val, Leu, Ile, Pro, Phe, Met, Trp, Cys or an amino acid having hydrophobicity equivalent to that of the foregoing amino acids, more preferably "Gly, Ala, Val, Leu, Ile, Pro, Phe, Met, Trp, or Cys"), and each parenthesized amino acid may be absent.

Preferably, the peptide of the present invention comprises at least an amino acid sequence represented by the following formula (4):
Xaa Xaa Xaa Asp Xaa1 Xaa1 Gly Phe Gly Xaa Xaa Xaa Xaa1 Xaa Xaa Xaa Xaa Asn (Xaa) (Gly) (Xaa) Xaa Xaa Xaa1 (Xaa) Xaa Xaa1 Xaa1 Xaa (Xaa) Xaa1 Xaa1 Xaa Xaa Xaa1 Xaa Xaa (Xaa) Xaa Xaa Gly Trp Xaa Xaa1 Xaa Xaa Xaa Xaa1 Arg Xaa Xaa1 Xaa1 Xaa Xaa Xaa Xaa (Xaa) [Formula (4)]
wherein each Xaa is an arbitrary amino acid, each Xaa1 is a hydrophobic amino acid, and each parenthesized amino acid may be absent.

More preferably, the peptide of the present invention comprises at least an amino acid sequence represented by the following formula (5):
Asp X11 X11 Gly Phe Gly Xaa Xaa Xaa X12 Xaa Xaa Xaa Xaa Asn (Xaa) (Gly) (Xaa) Xaa Xaa X13 (Xaa) Xaa X14 X15 Xaa (Xaa) X16 X11 Xaa Xaa X13 Xaa Xaa (Xaa) Xaa Xaa Gly Trp Xaa X11 Xaa Xaa Xaa X17 Arg Xaa X18 X19 [Formula (5)]
wherein each Xaa is an arbitrary amino acid, each X11 is Ile, Val or Leu, X12 is Ile or Val, each X13 is Phe or Leu, X14 is Pro, Ala, or Val, X15 is Pro, Ala, Val, or Met, X16 is Ala, Val or Leu, X17 is Pro, Val, Leu or Ile, X18 is Ala, Val, Leu, Ile or Met, and X19 is Ala, Val or Gly, and each parenthesized amino acid may be absent.

Further preferably, the peptide of the present invention comprises at least an amino acid sequence represented by the following formula (6):
Xaa Xaa Xaa Asp X11 X11 Gly Phe Gly Xaa Xaa Xaa X12 Xaa Xaa Xaa Xaa Asn (Xaa) (Gly) (Xaa) Xaa Xaa X13 (Xaa) Xaa X14 X15 Xaa (Xaa) X16 X11 Xaa Xaa X13 Xaa Xaa (Xaa) Xaa Xaa Gly Trp Xaa X11 Xaa Xaa Xaa X17 Arg Xaa X18 X19 Xaa Xaa Xaa Xaa (Xaa) [Formula (6)]
wherein each Xaa is an arbitrary amino acid, each X11 is Ile, Val or Leu, X12 is Ile or Val, each X13 is Phe or Leu, X14 is Pro, Ala, or Val, X15 is Pro, Ala, Val, or Met, X16 is Ala, Val or Leu, X17 is Pro, Val, Leu or Ile, X18 is Ala, Val, Leu, Ile or Met, and X19 is Ala, Val or Gly, and each parenthesized amino acid may be absent.

Particularly preferably, the peptide of the present invention comprises at least one or more amino acid sequences selected from the amino acid sequences of SEQ ID NOS: 3 to 9 represented by the following formulas (7) to (13):
Gly Val Ala Asp Leu Val Gly Phe Gly Thr Gly Gly Val Tyr Ile Ile Arg Asn Ser Leu Leu Ile Gln Val Val Lys Val Ile Asn Asn Phe Gly Tyr Asp Ala Gly Gly Trp Arg Val Glu Lys His Val Arg Leu Leu Ala Asp Thr Thr Gly Asp [Formula (7), SEQ ID NO: 3],
Asn Gln Ser Asp Val Val Gly Phe Gly Glu Asn Gly Val Trp Ile Ser Thr Asn Asn Gly Asn Asn Thr Phe Thr Asp Pro Pro Lys Met Val Ile Ala Asn Phe Ala Tyr Asn Ala Gly Gly Trp Arg Val Glu Lys His Ile Arg Phe Met Ala Asp Leu Arg Lys Thr [Formula (8), SEQ ID NO: 4],
Gly Arg Ala Asp Ile Val Gly Phe Gly Glu Ala Gly Ile Leu Val Ser Leu Asn Asn Gly Gly Ser Gln Phe Ala Pro Ala Gln Leu Ala Leu Asn Asn Phe Gly Tyr Ala Gln Gly Trp Arg Leu Asp Arg His Leu Arg Phe Leu Gly Asp Ile Thr Gly Asp (Formula (9), SEQ ID NO: 5],
Gly Leu Leu Asp Val Val Gly Phe Gly Glu Asn His Val Tyr Ala Ala Arg Asn Asn Gly Asn Gly Thr Phe Gln Pro Ala Gln Ala Val Val Asn Asn Phe Cys Val Gly Ala Gly Gly Trp Thr Ile Ala Ser His Pro Arg Val Ile Ala Asp Leu Thr Gly Asp [Formula (10), SEQ ID NO: 6],
Lys Arg Ala Asp Ile Leu Gly Phe Gly Gly Ala Gly Val Tyr Thr Ser Leu Asn Asn Gly Asn Gly Thr Phe Gly Ala Val Asn Leu Val Leu Lys Asp Phe Gly Thr Ala Ser Gly Trp Arg Val Glu Lys His Val Arg Cys Val Ala Pro Leu Thr Asn Lys [Formula (11), SEQ ID NO: 7],
Lys Val Gly Asp Ile Ile Gly Phe Gly Asp Ala Gly Val Tyr Val Ala Leu Asn Asn Gly Asn Gly Thr Phe Gly Pro Val Lys Arg Val Ile Asp Asn Phe Gly Tyr Asn Gln Gly Trp Arg Val Asp Lys His Pro Arg Phe Val Val Asp Leu Thr Gly Asp [Formula (12), SEQ ID NO: 8], and
Gly Cys Ala Asp Ile Val Gly Phe Gly Glu Asn Ser Val Trp Ala Cys Met Asn Lys Gly Asp Gly Thr Phe Gly Pro Met Met Lys Leu Ile Asp Asp Leu Thr Val Ser Lys Gly Trp Thr Leu Gln Arg Thr Val Arg Tyr Ala Ala Asn Leu Tyr Leu [Formula (13), SEQ ID NO: 9].

Further particularly preferably, the peptide of the present invention comprises all of the amino acid sequences of SEQ ID NOS: 3 to 9 represented by the foregoing formulas (7) to (13) by one for each.

In the above formulas, when a plurality of the same symbols (Xaa1, X11 to X19 etc.) are present in the same formula, they may be identical or different from each other or one another.

The polypeptide of the present invention mentioned above is usually a lectin having activity for specifically binding to a sugar chain (preferably a sugar chain having an N-acetylglucosamine residue, more preferably a sugar chain having an N-acetylglucosamine residue at its end, particularly preferably an N-acetylglucosamine residue of a sugar chain having the N-acetylglucosamine residue at its end, extremely preferably an N-acetylglucosamine residue of a sugar chain having GlcNAc → Man containing the N-acetylglucosamine residue at its end), more preferably lectin having activity for specifically binding to an N-acetylgiucosamine residue.

Further, the polypeptide of the present invention include a polypeptide of lectin which comprises at least a part of the amino acid sequence shown in SEQ ID NO: 2, and may have substitution, deletion or insertion of one or more amino acid residues that does not substantially spoil its binding activity to a sugar chain (preferably a sugar chain having an N-acetylglucosamine residue, more preferably a sugar chain having an N-acetylglucosamine residue at its end, particularly preferably an N-acetylglucosamine residue of a sugar chain having the N-acetylglucosamine residue at its end, extremely preferably an N-acetylglucosamine residue of a sugar chain having GlcNAc → Man containing the N-acetylglucosamine residue at its end). The polypeptide of the present invention is preferably a polypeptide of lectin which comprises at least a part of the amino acid sequence shown in SEQ ID NO: 2, and does not have substitution, deletion or insertion of an amino acid residue or residues.

Such substitution, deletion or insertion of an amino acid residue or residues may be obtained by expression of a DNA which is obtainable by introducing such substitution, deletion or insertion of a nucleotide or nucleotides that causes the substitution, deletion or insertion of an amino acid residue or residues into a DNA encoding at least a part of the amino acid sequence shown in SEQ ID NO: 2. The substitution, deletion or insertion of a nucleotide or nucleotides can be introduced into a DNA sequence by preparing a sequence having restriction enzyme cleavage sites at the both ends and the both sides of the mutation region, and replacing the corresponding portion of an unmutated DNA sequence with it. The substitution, deletion or insertion can also be introduced into a DNA sequence by the site-specific mutagenesis (Kramer, W. and Frits, H.J., Methods in Enzymol., 154, 350 (1987); Kunkel, T.A. et al., Methods in Enzymol., 154, 367 (1987)) or the like.

The sugar chain-binding activity of the polypeptide of lectin (lectin activity) can be measured, for example, by the lectin activity measurement method which will be described hereinafter, and those skilled in the art will readily recognize substitution, deletion or insertion of one or more amino acid residues that does not substantially spoil the binding activity to the sugar chain.

It is also possible to alter a part of the structure of the polypeptide by naturally-occurring or artificial mutation without substantially changing the sugar chain-binding activity. The polypeptide of the present invention includes a polypeptide having a structure corresponding to an homogeneous variant of a polypeptide having at least a part of the amino acid sequence shown in SEQ ID NO: 2.

In the present specification, the expression of "to comprise at least a part of the amino acid sequence shown in SEQ ID NO: 2" means that a sequence has a minimum and essential amino acid sequence of polypeptide required to have the sugar chain-binding activity.

The polypeptide of the present invention further include a polypeptide comprising a portion of the polypeptides mentioned above. The term "portion" preferably means a portion having a certain activity or function, for example, having the sugar chain-binding activity, having antigenicity, or the like. Those skilled in the art will readily recognize such a portion.

As the polypeptide of the present invention, a polypeptide having the amino acid sequence represented by the amino acid numbers 18-402 in SEQ ID NO: 2 is preferred, and a polypeptide having the amino acid sequence represented by the amino acid numbers 1-402 in SEQ ID NO: 2 is particularly preferred.

The polypeptide of the present invention is not necessary to be present alone, and may be a part of a fusion polypeptide (fusion protein).

As another polypeptide constituting the fusion polypeptide, for example, polypeptides that can be biotinylated, glutathione-S-transferase or a part thereof, a protein encoded by the gene 10 of T7 phage (gene 10 product of T7 phage) or a part thereof and the like can be mentioned.

While the aforementioned polypeptide of the present invention may be chemically synthesized based on its sequence elucidated by the present invention, it is possible and preferable to obtain it by expression of the DNA of the present invention, which will be described hereinafter. That is, the polypeptide of the present invention can be produced by culturing cells harboring the DNA of the present invention in a suitable culture medium so that the polypeptide of the present invention should be produced and accumulated in the medium, and collecting the polypeptide of the present invention from the culture medium. While any host-vector systems usually used for the production of proteins can be used for the expression of the polypeptide of the present invention utilizing host cells harboring the DNA of the present invention, use of *Escherichia coli* K-12 or a mutant thereof and an expression vector (PinPointXa, pGEMEX, pET-5 (Promega), pGEX-5X (Pharmacia) etc.) is preferred.

Further, it is also possible to express the polypeptide of the present invention from the DNA of the present invention by using pPIC3K (Invitrogen), which is an *E. coli*/yeast shuttle vector, and yeast such as *Pichia pastoris* as a host.

The polypeptide may either be directly expressed by using the DNA of the present invention, or may be expressed as a fusion protein. The DNA of the present invention may be expressed in its full length, or its part may be expressed to obtain a partial peptide.

It should be noted that, while it has been known that sugar chain structure abnormality in IgG, i.e., increase of sugar chains lacking galactose, is observed in rheumatoid arthritis (RA) patients, PVL specifically recognizes N-acetylglucosamine residue (GlcNAc) β1→2 mannose residue (Man) exposed in the above galactose-lacking IgG (agalacto-IgG). Therefore, it is also possible to prepare an *in vitro* diagnostic agent for detecting the agalacto-IgG by ELISA or the like by utilizing the polypeptide, the partial polypeptides thereof, or the fusion protein comprising the polypeptide and another protein according to the present invention prepared as described above, which has the same activity as PVL.

The antibody of the present invention is an antibody which binds to the polypeptide of the present invention, and may be polyclonal or monoclonal. The antibody of the present invention can be prepared in a conventional manner by utilizing the polypeptide, or the partial polypeptide thereof, or the fusion protein comprising the polypeptide and another protein according to the present invention as described above, as antigen, for example, as follows.

The antibody of the present invention which is polyclonal can be obtained by immunizing an animal to be immunized such as mouse, rat, guinea pig, rabbit, goat, sheep or the like with the above antigen, and collecting serum from the animal. An adjuvant is preferably used for immunization of the animal, because it activates antibody-producing cells. The resulting anti-serum may be purified by a conventional method to obtain immunoglobulin fractions.

The antibody of the present invention which is monoclonal can be obtained, for example, as follows. The aforementioned antigen is administered to abdominal cavity, subcutis, footpad or the like of an animal to be immunized such as mouse, rat, guinea pig, rabbit, goat, sheep, or the like, and then spleen or popliteal lymph node is extracted. Cells obtained from these organs are fused with myeloma cells, which are cells of a tumor cell line, to establish hybridomas. The resulting hybridomas are continuously proliferated, and cell lines continuously producing antibodies specifically binding to the polypeptide of the present invention are selected from the resulting hybridomas. By culturing the selected cell lines in a suitable medium, the antibody of the present invention which is monoclonal can be obtained in the culture medium. Alternatively, a large amount of the antibody of the present invention which is monoclonal can be prepared by culturing the hybridomas in living bodies such as in mouse abdominal cavity. As the cells used for the cell fusion, lymph node cells, lymphocytes in peripheral blood and the like can also be used in addition to spleen cells. The myeloma cell lines are preferably those derived from cell species allogeneic to the immunized animal rather than those derived from heterologous cell species, and such allogenic cells can give stable antibody-producing hybridomas.

Examples of the method for purifying the resulting polyclonal and monoclonal antibodies include, for instance, salting-out with sodium sulfate, ammonium sulfate or the like, selective precipitation separation methods by low temperature alcohol precipitation, precipitation with polyethylene glycol, isoelectric precipitation and the like, electrophoresis, ion exchange chromatography utilizing ion exchanger such as DEAE (diethylaminoethyl) derivatives and CM (carboxymethyl) derivatives, affinity chromatography utilizing protein A or protein G, hydroxyapatite chromatography, immunoadsorption chromatography utilizing immobilized antigens, gel filtration, ultracentrifugation and the like. The antibody of the present invention may be a fragment comprising the antigen-binding site Fab obtained by treating the whole antibody with a protease not digesting the antigen-binding site (Fab) such as plasmin, pepsin and papain. If a nucleotide sequence of gene encoding the antibody of the present invention or an amino acid sequence of the antibody is determined, fragments comprising Fab of the antibody of the present invention or chimeric antibodies such as chimeric antibodies comprising Fab region of the antibody of the present invention can be genetically engineered, and such fragments and chimeric antibodies are encompassed within the antibody of the present invention.

### 〈6〉 DNA of the present invention

The DNA of the present invention includes a DNA encoding the lectin having the following properties:
(1) binding to a sugar chain (preferably a sugar chain having an N-acetylglucosamine residue, more preferably a sugar chain having an N-acetylglucosamine residue at its end, particularly preferably an N-acetylglucosamine residue of a sugar chain having the N-acetylglucosamine residue at its end, extremely preferably an N-acetylglucosamine residue of a sugar chain having GlcNAc → Man containing the N-acetylglucosamine residue at its end),
(2) having a molecular weight of about 40 kilodalton as estimated by SDS-polyacrylamide gel electrophoresis,
(3) having the aforementioned particular amino acid composition, and
(4) present in fruiting body of *Psathyrella velutina*.

The DNA of the present invention include a DNA encoding at least a part of the aforementioned polypeptide of the present invention.

The lectin mentioned above is preferably composed of 402 amino acid residues in addition to the aforementioned properties.

The nucleotide sequence of the DNA of the present invention is not particularly limited so long as it encodes at least a part of the lectin having the aforementioned properties or those having equivalent properties.

The DNA of the present invention include all of DNAs at least encoding the polypeptide of the present invention described above. For example, the DNA of the present invention include DNAs encoding a polypeptide of lectin which comprises at least a part of the amino acid sequence shown in SEQ ID NO: 2, and may have substitution, deletion or insertion of one or more amino acid residues that does not substantially spoil its binding activity to a sugar chain (preferably a sugar chain having an N-acetylglucosamine residue, more preferably a sugar chain having an N-acetylglucosamine residue at its end, particularly preferably an N-acetylglucosamine residue of a sugar chain having the N-acetylglucosamine residue at its end, extremely preferably an N-acetylglucosamine residue of a sugar chain having GlcNAc → Man containing the N-acetylglucosamine residue at its end), a polypeptide of lectin which comprises at least a part of the amino acid sequence shown in SEQ ID NO: 2, a polypeptide comprising a portion of the foregoing polypeptides, and the like.

As a specific example of the DNA of the present invention, a DNA having a nucleotide sequence encoding the amino acid sequence represented by the amino acid numbers 18-402 or 1-402 in SEQ ID NO: 2 can be mentioned, and is preferred. More specifically, a DNA having a part or all of the nucleotide sequence shown in SEQ ID NO: 1 (nucleotide numbers 92-1246 or 41-1246) can be mentioned as an example of the DNA of the present invention, and is particularly preferred.

It will be readily understood by those skilled in the art that DNAs having a different nucleotide sequence due to the degeneracy of the genetic code are encompassed within the DNA of the present invention. Any of such DNAs fall within the scope of the DNA of the present invention.

While it is expected that a PVL gene derived form a chromosome may contain introns in the coding region, DNA fragments divided with such introns are also encompassed within the DNA of the present invention so long as they encode at least a portion of the polypeptide of PVL. That is, the term "to encode" used in the present specification also means to contain a nucleotide sequence which undergoes processing upon transcription and can eventually give a desired polypeptide.

The expression "to encode at least a part of polypeptide" used herein means to encode a portion having a certain activity or function, for example, having binding activity to a sugar chain (preferably a sugar chain having an N-acetylglucosamine residue, more preferably a sugar chain having an N-acetylglucosamine residue at its end, particularly preferably an N-acetylglucosamine residue of a sugar chain having the N-acetylglucosamine residue at its end, extremely preferably an N-acetylglucosamine residue of a sugar chain having GlcNAc → Man containing the N-acetylglucosamine residue at its end), having antigenicity, or the like, or a portion whose corresponding nucleotide sequence is specific for the PVL gene, and hence can be used as a primer or probe.

The present invention include DNA or RNA which is complementary to the DNA of the present invention. The DNA of the present invention may be single-stranded and composed only of a coding strand encoding the lectin, or it may be double-stranded, and composed of the foregoing single strand and a DNA or RNA strand having a complementary sequence thereto.

The DNA of the present invention may contain the full length of the coding region encoding the full length PVL, or it may encode a partial peptide of PVL.

Since the nucleotide sequence of the DNA of the present invention has been elucidated by the present invention, the DNA of the present invention can be obtained by chemical synthesis based on the sequence, or by amplification from a *Psathyrella velutina* chromosome DNA or mRNA by PCR (polymerase chain reaction) using oligonucleotide primers which are prepared based on the sequence. The DNA of the present invention was originally obtained by cDNA cloning comprising the following steps as detailed in the examples hereinafter:
① collection of Psathyrella velutina and extraction of total cell mRNA from *Psathyrella velutina* fruiting body;
② purification of PVL from *Psathyrella velutina* fruiting body and preparation of anti-PVL polyclonal antibody;
③ determination of partial amino acid sequence of the purified PVL;
④ preparation of poly(A)+RNA from *Psathyrella velutina* cells and construction of *Psathyrella velutina* cDNA library;
⑤ screening of the *Psathyrella velutina* cDNA library with the antibody and selection of PVL full length cDNA;
⑥ determination of full length nucleotide sequence and comparison of PVL amino acid sequence deduced from the nucleotide sequence and the PVL partial amino acid sequence; and
⑦ construction of PVL expression plasmid and verification of lectin activity of the expressed polypeptide.

However, the production method of the DNA of the present invention is not limited to the above method, and the DNA of the present invention can also be produced by screening of a cDNA library with a DNA probe which is synthesized based on a partial amino acid sequence, expression cloning based on a lectin activity, and other known cDNA cloning techniques.

An example of the method for obtaining the DNA of the present invention will be specifically explained hereinafter.

### (1) Purification of PVL from Psathyrella velutina fruiting body and preparation of anti-PVL polyclonal antibody

### ① Purification of PVL

PVL can be purified from *Psathyrella velutina* fruiting body by a combination of usual purification methods for proteins. Specifically, it is preferably performed according to the method described in J. Biol. Chem., 264, 173-177 (1989).

The sugar chain-binding activity of PVL (lectin activity) can be measured by ELISA utilizing biotiylated PVL according to Kochibe et al. (J. Biol. Chem., 264, 173-177 (1989)), or by the agalacto-IgG binding method of Tsuchiya et al. (J. Immunology, 151, 1137-1146 (1993)). The sugar chain-binding activity of lectin can also be measured by the hemagglutination method and the hemagglutination inhibition method described in the examples hereinafter.

The presence of naturally-occurring PVL and a recombinant PVL polypeptides can be detected by Western blotting utilizing the anti-PVL antibody described hereinafter.

### ② Preparation of rabbit anti-PVL polyclonal antibody

An anti-PVL antibody can be prepared by immunizing rabbit with PVL according to a known method. General reviews of immunization and detection are detailed in, for example, Ed Harlow and David Lane (Antibodies, A Laboratory Manual, Cold Spring Harbor Laboratory, 1988).

### (2) Preparation of Psathyrella velutina cDNA

### ① Preparation of total RNA

The total RNA can be obtained by a known method (Kingston, R. E., (1991) in Current Protocols in Molecular Biology, Suppl. 14, Unit 4.2, Greene Publishing Associates and Wiley Interscience, New York etc.). While the material is not limited so long as it expresses mRNA of PVL, fresh *Psathyrella velutina* fruiting body is preferred to obtain full length mRNA. Because there may be regional variants or subspecies, *Psathyrella velutina* growing on the ground of Mt. Haruna or Mt. Akagi, Gunma-ken at an altitude of 500 to 600 m is preferred among *Psathyrella velutina* species.

The total RNA can be obtained from *Psathyrella velutina* fruiting body by commonly-used preparation methods of total RNA as described above, but it is preferably prepared by the guanidine thiocyanate/CsCl method (Kingston, R. E., (1991) in Current Protocols in Molecular Biology, Suppl. 14, Unit 4.2, Greene Publishing Associates and Wiley Interscience, New York).

### ② Preparation of poly(A)+RNA

While poly(A)+RNA may be purified from the total RNA obtained as described above by a known method such as oligo-(dT) cellulose column chromatography, it is preferable to use a mRNA separation system utilizing magnetism, for example, PolyATtract mRNA isolation system (Promega).

### ③ Synthesis of Psathyrella velutina cDNA and construction of expression library

cDNA can be synthesized by a reverse transcriptase reaction utilizing poly(A)+RNA as a template. It is convenient to use a commercially available kit for cDNA synthesis. By using RiboClone cDNA synthesis systems (Promega), for example, cDNA can be synthesized, and ligated to a cloning vector such as λgt11 digested with *Eco*RI. In the present invention, it is preferable to use λgt11 digested with *Eco*RI. As the primer for the reverse transcriptase reaction, oligo(dT)n can be used, but it is preferable to use a random oligonucleotide primer.

By using a λ phage expression vector such as λgt11, a recombinant DNA can be directly introduced into *E. coli* treated with calcium chloride, but a method for more efficiently infecting *E. coli* has commonly been used in which the DNA is preliminarily encapsulated in phage outer coat *in vitro* (called *in vitro* packaging), and introduced into *E. coli*, and kits therefor are commercially available (Gigapack II packaging extract, Stratagene etc.). This method is preferably used for the present invention. By this method, a *Psathyrella velutina* cDNA library can be constructed.

*E. coli* is infected with the recombinant DNA undergone *in vitro* packaging, but a specific *E. coli* strain must be selected depending on the kind of cloning vector to be used. For example, when λgt11 is used as the cloning vector, an *E. coli* strain which does not express β-galactosidase activity, and hence is suitable as a phage host, such as *Escherichia coli* Y1088, may be selected. When λgt11 is used as the vector, it may be suspended in soft agar medium with indicator bacterial cells, and overlaid on agar medium to form plaques.

### ④ Cloning of PVL cDNA

In the aforementioned λgt11, the *Psathyrella velutina* cDNA is inserted in the restriction enzyme *Eco*RI cleavage site in the downstream of the β-galactosidase gene. Therefore, the *Psathyrella velutina* gene fragment fuses with the β-galactosidase gene, and a *Psathyrella velutina*-derived protein is expressed.

Then, phage clones having cDNA of PVL can be selected from the cDNA library obtained as described above based on the reactivity with an anti-PVL antibody. The preparation and selection of phage clones which react with the anti-PVL antibody may be performed by a usual method, for example, according to the description of Sambrook (Sambrook, J. et al, Molecular cloning, Cold Spring Harbor lab. Press. 1989). For example, ProtBlot II AP system (Promega) is preferably used for the detection of the phage clones.

Phage DNAs can be prepared from the selected positive clones, and digested with a suitable restriction enzyme to excise candidate cDNAs. The resulting cDNAs are determined for their nucleotide sequences as they are, or after subcloned into a suitable plasmid.

The region of the open reading frame of the cDNA nucleotide sequence encoding PVL, which was determined as described above, is shown in SEQ ID NO: 1, and the corresponding amino acid sequence is shown in SEQ ID NO: 2. A protein composed of 402 amino acid residues and having a molecular weight of 43 kilodalton (kDa) is expected from the single open reading frame beginning with the first ATG codon.

As for the DNA obtained as described above, a polypeptide encoded by the DNA may have substitution, deletion or insertion of one or more amino acid residues, provided that the binding activity to a sugar chain (preferably a sugar chain having an N-acetylglucosamine residue, more preferably a sugar chain having an N-acetylglucosamine residue at its end, particularly preferably an N-acetylglucosamine residue of a sugar chain having the N-acetylglucosamine residue at its end, extremely preferably an N-acetylglucosamine residue of a sugar chain having GlcNAc → Man containing the N-acetylglucosamine residue at its end) is not substantially spoiled. The substitution, deletion or insertion of a nucleotide or nucleotides can be introduced into a DNA sequence by preparing a sequence having restriction enzyme cleavage sites at the both ends and the both sides of the mutation region, and replacing the corresponding portion of an unmutated sequence with it. The substitution, deletion or insertion of a nucleotide or nucleotides can also be introduced by the site-specific mutagenesis (Kramer, W. and Frits, H.J., Methods in Enzymol., 154, 350 (1987); Kunkel, T.A. et al., Methods in Enzymol., 154, 367 (1987)) or the like.

The sugar chain-binding activity of the polypeptide can be measured, for example, by the lectin activity measurement method mentioned above, and those skilled in the art will readily recognize substitution, deletion or insertion of one or more amino acid residues that does not substantially spoil the binding activity to a sugar chain.

### (3) Sequence determination of PVL clone

### ① Determination of partial amino acid sequence of PVL

While the method for fragmentation of the purified PVL is not particularly limited, a protease for sequence analysis such as Lysyl Endopeptidase (Wako Pure Chemical Industries Co., Ltd.) is preferably used. The excised gel may be brought into contact with a protease, and then separated by SDS-PAGE or gel filtration. As a simple procedure, the method described in Cleveland, D. W., Fischer, S. G., Kirshner, M. W., and Laemmli, U. K.(1977) J. Biol. Chem. 252, 1102-1106 can be mentioned. In this method, an excised protein band is inserted into a well of another gel, SDS-PAGE is performed while a buffer containing a protease is placed on the inserted gel, then the migration is temporarily stopped by shutting down the electric source before the edge of the dye enters into the separation gel to allow enzymatic digestion for about 30 minute, and then electrophoresis is started again. This method is convenient since it enables the enzymatic digestion and separation of peptide fragments after the digestion by one step. The peptides formed by the protease digestion are transferred to a PVDF membrane, a nitrocellulose membrane or the like. This membrane is stained with a dye staining proteins such as Coomassie Brilliant Blue and Amido Black, and then the peptide bands are excised. As for the peptides formed after the protease digestion contained in the PVDF membrane or the nitrocellulose membrane, amino-terminal sequences of the peptides can be determined by a known method. By determining partial sequences of the peptides, consistency with the nucleotide sequences can be confirmed.

### ② Expression of PVL gene

The PVL gene can be expressed in a well known manner by using a currently used or commercially available expression vector for a host of *E. coli*, yeast or the like. The expression vector may be either one for directly expressing the PVL DNA from the initiation codon, or one for expressing it as a fusion protein. As the expression vector, PinPointXa, pGEMEX, pET-5 (Promega), pGEX-5X (Pharmacia), pPIC3K (Invitrogen) which is a *E. coli* /yeast shuttle vector, and the like are preferably used.

The recombinant DNA of the present invention can be obtained by introducing the DNA of the present invention into a DNA encoding another arbitrary polypeptide in a conventional manner. The DNA encoding another arbitrary polypeptide preferably has a nucleotide sequence encoding a peptide which can be biotinylated.

The recombinant vector containing the DNA of the present invention can also be obtained by introducing the DNA of the present invention into an arbitrary vector in a conventional manner. The vector is preferably an expression vector. While the expression vector is not particularly limited so long as it is a vector which can commonly be used in the genetic engineering field, it preferably has a nucleotide sequence encoding a peptide which can be biotinylated, glutathione-S-transferase, gene 10 product of T7 phage or the like.

If the DNA of the present invention is introduced by using the expression vector so that a fusion polypeptide containing a peptide which can be biotinylated should be formed, for example, the polypeptide to be formed in a host cell would be able to be easily purified by using an avidin-bound resin or the like. Further, if the DNA of the present invention is introduced by using the expression vector so that a fusion polypeptide containing glutathione-S-transferase should be formed, for example, the polypeptide to be formed in a host cell would be able to be easily purified by using a glutathione-bound resin or the like.

A transformant cell can be obtained by introducing the recombinant vector mentioned above into a host cell by a conventional method. The host cell is preferably an *E. coli* or yeast cell. A suitable combination of the recombinant vector and the host cell can be suitably selected by those skilled in the art.

### Examples

The present invention will be further explained more specifically with reference to the following examples. However, these examples are mentioned only for illustration, and the present invention is not limited to them. The methods commonly used in the examples will be explained first. The term "percent (%)" means "percent by weight" unless otherwise indicated.

### Assay of lectin activity

Assays of lectin activity and binding specificity of PVL were performed by the method of Kochibe et al. (Kochibe, A. and Matta, K., J. Biol. Chem., 264, 173-177 (1989)). More specifically, the assays were performed by the following methods.

### Hemagglutionation method:

By using a 96-well microtiter plate, 25 µl of a solution of the protein serially diluted with PBS and 25 µl of 2% suspension of type O blood human erythrocytes in PBS are mixed. The minimum amount of PVL causing agglutination when the mixture is left at room temperature for 20 minutes is determined as 1 agglutination unit of PVL.

### Hemagglutionation inhibition method:

25 µl of a serially diluted inhibitor solution having a known concentration and 8 agglutination units of PVL (25 µl) are mixed, left at 4°C for 2 hours, and then 50 µl of 2% suspension of type O blood human erythrocytes in PBS was added. The concentration (mM) of the inhibitor completely inhibits the agglutination after the mixture is left at room temperature for 20 minutes is defined as the inhibition concentration.

### Example 1: Purification of PVL from Psathyrella velutina fruiting body and production of anti-PVL polyclonal antibody

### 1. Preparation of anti-PVL antibody

*Psathyrella velutina* mushrooms growing on the ground of Mt. Akagi, Gunma-ken at an altitude of 500-600 m were collected, and about 40mg of PVL, which showed substantially uniform electrophoresis band, was obtained from 300 g of fruiting body of the *Psathyrella velutina* according to the method described in J. Biol. Chem., 264, 173-177 (1989).

To produce an anti-PVL antibody, three 15-week old rabbits were immunized. As the first immunization, an emulsion containing about 2 mg of PVL in Freund Complete Adjuvant was subcutaneously injected to the rabbits on their back. Then, an emulsion formed by mixing the same amount of PVL with Freund Incomplete Adjuvant was intramuscularly injected once a week for three weeks, and the rabbits were exsanguinated one week later. The resulting rabbit blood was left stand at 4°C overnight to precipitate blood clots, and then centrifuged at 2000 x g at 4°C for 15 minutes to obtain serum. Reactivity of the obtained serum with PVL was confirmed by the Ouchterlony's diffusion method, and IgG was purified from the antiserum by using a Protein A affinity column (Amersham LIFE SCIENCE).

### 2. Assay of anti-PVL polyclonal antibody by ELISA

100 µl of 17 µg/ml PVL solution (25 mM NaHCO₃/NaOH buffer, pH 9.4) was introduced into each well of a 96-well plate (Nunc), and left at 37°C for 95 minutes, and then each well was washed once with 145 mM NaCl, 5 mM phosphate buffer (PBS, pH 7.5). 150 µl of 1% fetal bovine serum albumin (BSA-Fraction V, sold by Seikagaku Corporation) was added to each well, and blocking was performed at 37°C for one hour. Each well was washed once with PBS, and 100 µl of diluted anti-PVL antibody was added thereto, and incubated at 37°C for 1.5 hours. Each well was washed three times with PBS containing 0.1% Tween-20 (PEST), and 100 µl of alkaline phosphatase-conjugated anti-rabbit IgG +IgM goat antibody (Cosmo Bio Co., Ltd) was added thereto, and incubated at 37°C for one hour. Each well was washed three times with PBST, and 100 µl of a substrate solution (1 mg/ml SIGMA 104 PHOSPHATASE SUBSTRATE, Sigma) was added thereto, and left at room temperature for five minutes. Then, absorbance of the color-developed solution at a wavelength of 405 nm (reference wavelength; 630 nm, A405/630) was determined by Well Reader SK601 (sold by Seikagaku Corporation).

### 3. Western blotting utilizing anti-PVL antibody

Sodium dodecyl sulfate (SDS)-polyacrylamide gel electrophoresis (SDS-PAGE) was performed by partially modifying the method of Laemmli et al. (Laemmli, UK., (1970), Nature 227, 680-685). Each sample composed of a sample buffer (50 mM Tris-HCl, 2% SDS, 20% glycerol, 0.002% Bromophenol Blue (BPB), 5% β-mercaptoethanol) and a sample in equal volumes was subjected to electrophoresis using 12.5% polyacrylamide gel at 20 mA for about 2 hours. Transfer from the gel after electrophoresis to a PVDF membrane (Immobilon, Millipore) was performed according to the method of Nielsen PJ. and Towbin H. (Nielsen PJ. and Towbin H., (1982), J. Biol. Chem., 257, 12316-12321), which was partially modified. The PVDF membrane was immersed in 100% methanol for 20 seconds, washed with distilled water for several seconds, and immersed in 25 mM Tris/192 mM glycine, 20% methanol (blotting buffer) for five minutes. Filter paper sheets (3MM chromatography paper, Whatman) immersed in the blotting buffer were placed on the both sides of the stacked membrane and gel, and applied with an electric current of 180 mA for one hour. After the transfer, the PVDF membrane was washed 3 times with TBST (10 mM Tris-HCl (pH 7.5), 150 mM NaCl, 0.1% Tween 20) for five minutes each. The membrane was transferred into TBST containing 3% BSA, and blocked for one hour. Then, the membrane was washed again three times with TBST for five minutes each, transferred into TBST containing the anti-PVL antibody, and left at room temperature for one hour with shaking. The membrane was washed with TBST in the same manner, transferred into TBST containing an alkaline phosphatase-conjugated anti-rabbit IgG (Fc) antibody (Promega), and left at room temperature for one hour with shaking. Then, the membrane was washed with TBST for 5 minutes, further washed twice with TBS (TBST not containing Tween 20) for five minutes each, and transferred into Western Blue substrate (Promega) for color development. After appropriate color development was obtained, the membrane was washed with distilled water to terminate the color development.

### 4. Assay of anti-PVL antibody

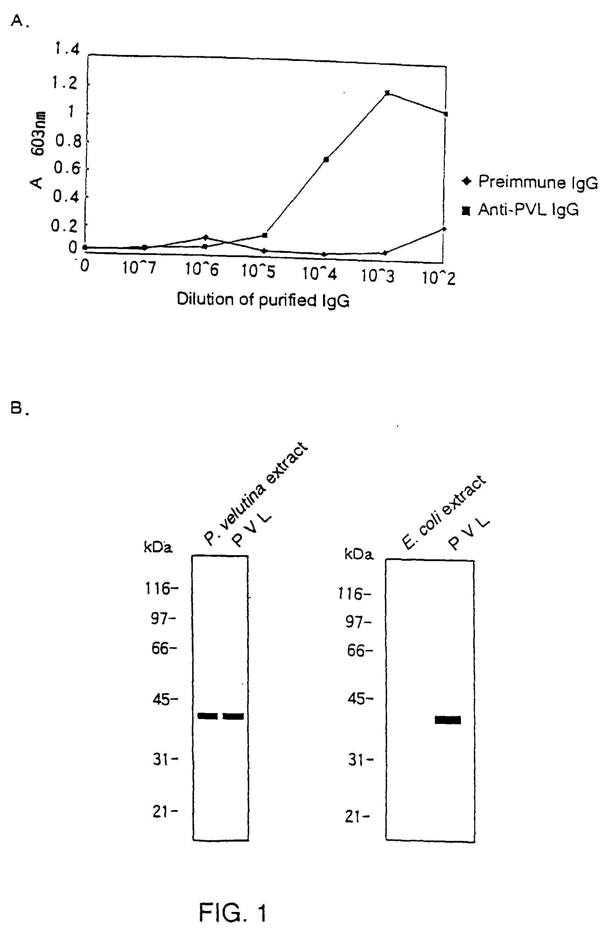

ELISA and Western blotting were performed for assaying the purified anti-PVL antibody (Figure 1). A 10000-fold dilution could be used for ELISA. In the assay by Western blotting, any bands other than that of PVL at 40 kDa were not identified in the extract of *Psathyrella velutin*a. Any bands were not confirmed also in the assay utilizing an extract derived from *E. coli* K12 strain, and it was considered that there were no cross-reactive proteins.

### 5. Determination of PVL partial amino acid sequence

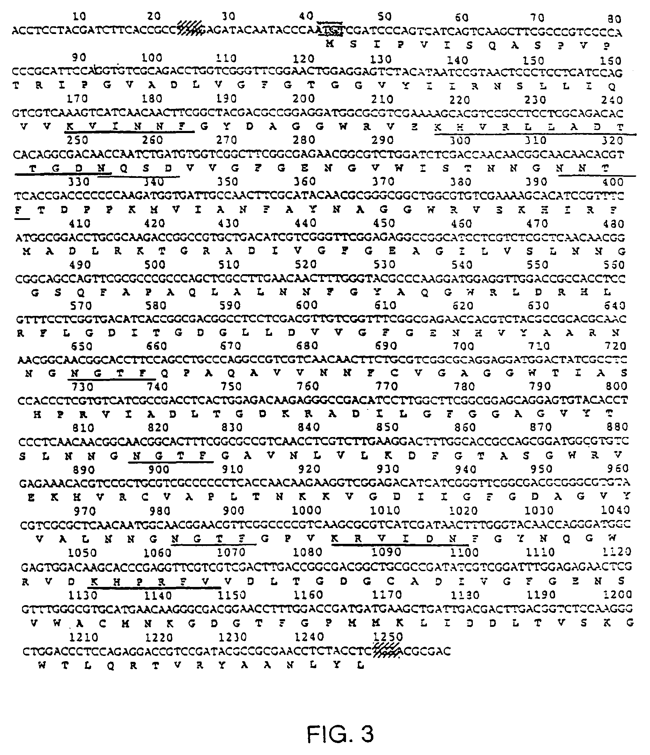

The purified PVL was fragmented by using Lysyl Endopeptidase (Wako Pure Chemical Industries Co., Ltd.). The fragmented polypeptides were separated by gel filtration. Partial amino acid sequences of four peptide fragments were determined. These agreed with the amino acid sequences deduced from the nucleotide sequences as described hereinafter (Figure 3).

### Example 2: Preparation of Psathyrella velutina cDNA

### 〈1〉 Construction of Psathyrella velutina fruiting body λ gt11 cDNA library

*Psathyrella velutina* fruiting body was collected from *Psathyrella velutina* mushroom growing on the ground of Mt. Akagi, Gunma-ken at an altitude of 500 to 600 m. The fresh *Psathyrella velutina* fruiting body was ground by a Polytron homogenizer in the presence of a protein-denaturation agent, and the total cell RNA was extracted. After ethanol precipitation, the total cell RNA was stored at -80°C.

Purification of mRNA from the total RNA was performed by using PoLyATract mRNA isolation system (Promega) according to the protocol of the attached instructions. Synthesis of cDNA from the purified mRNA was carried out by using RiboClone cDNA Synthesis Systems AMV RT (a kit available from Promega) and reagents attached to the kit. The mRNA was added to 50 mM Tris-HCl (pH 8.5), 50 mM KCl, 10 mM MgCl₂, 500 µM spermidine, 10 mM dithiothreitol (DTT), and 1 mM dNTPs, and 1 µg of random primers was added thereto, and then 25 unit (U) of RNasin, 4 mM sodium phosphate, and 30U AMV RT (Avian Myeloblastosis Virus Reverse Transcriptase) were added, and the mixture was incubated at 37°C for one hour. Thereafter, to the reaction mixture, as final concentrations, 40 mM Tris-HCl (pH 7.2), 90 mM KCl, 3 mM MgCl₂, 3 mM DTT, and 50 µg/ml BSA were added, and then 0.8 U of RNase H, and 23 U of DNA polymerase I were added, and the mixture was incubated at 14°C for two hours. Then, cDNA was denatured by placing the reaction mixture at 70°C for 10 minutes, and 2U of T4 DNA polymerase was added thereto, and the mixture was incubated at 37°C for ten minutes. Thereafter, 20 mM ethylenediaminetetraacetic acid (EDTA) as a final concentration was added to the reaction mixture to terminate the reaction. After phenol extraction and ethanol precipitation, the resulting product was dissolved in 10 mM Tris-HCl (pH 8.0), 1 mM EDTA (TE buffer). *Eco*RI adapter (Promega) was ligated to the cDNA, and then ligated to λ gt11 vector arm (Promega). The resulting DNA was packaged by using GigapackIII Gold Packaging Extract (Stratagene), and the phage was infected into *E. coli* Y1090, and collected to prepare a λgt11 library.

### 〈2〉 Separation of PVL cDNA

### 1. Screening of cDNA library utilizing anti-PVL antibody

Y1090 cells cultured overnight in LB (Luria Broth) medium were infected with 1.5 x 10⁴ of λgt11 cDNA library, then inoculated onto a 15 mm plate, and incubated at 42°C for 3.5 hours. A nitrocellulose membrane (Millipore) preliminarily immersed in 10 mM isopropyl-β-D-thiogalactopyranoside (also referred to as "IPTG" hereinafter) for 30 minutes was carefully overlaid on the plate, and further incubated at 37°C for 3.5 hours. The nitrocellulose membrane was carefully peeled off from the plate, and washed twice with TBST. The membrane was transferred into TBST containing 1% BSA, and blocked at room temperature for one hour. The membrane was washed once with TBST, and reacted with the anti-PVL antibody at room temperature for 30 minutes. The membrane was washed three times with TBST, incubated in a solution containing an alkaline phosphatase-conjugated anti-rabbit IgG antibody (Promega) at room temperature for 30 minutes, washed three times with TBST, and further washed once with TBS. Color development reaction was performed in an alkaline phosphatase (AP) solution (100 mM Tris-HCl (pH 9.5), 100 mM NaCl, 5 mM MgCl₂) containing Nitroblue tetrazolium (NBT) and bromochloroindoyl phosphate (BCIP). When appropriate color development was obtained, the nitrocellulose membrane was transferred into a solution containing 20 mM Tris-HCl (pH 8.0), 5 mM EDTA to terminate the color development reaction. Phages were extracted from the resulting positive plaques, and infected into Y1090 cells to purify the positive clones by repeating the same procedure as described above.

### 2. Isolation of PVL cDNA clone

The prepared *Psathyrella velutina* cDNA library was screened, and six positive plaques were detected and purified from plaques of 1.4 x 10⁵ plaque forming units (pfu). cDNA was isolated from each clone, and digested with *Eco*RI, and molecular weight of the inserts was determined by agarose gel electrophoresis. The clones having a longest insert were λPVL231 and λPVL2111, which contained inserts having approximately the same length of about 1300 bp. The lengths of the inserts of λPVL511, λPVL3121, and λPVL2221 were approximately 700 bp, 300 bp, and 250 bp, respectively. As for λPVL611, no band considered to correspond to the insert could be confirmed by the electrophoresis, and the insert was considered to have a length of 100 bp or less. Because PVL has a molecular weight of 40 kDa (Kochibe, A. and Matta, K. (1989), J. Biol. Chem., 264, 173-177), the length of the expected open reading frame (ORF) for PVL is considered to be about 1200 bp. Therefore, it was estimated that λPVL231 and λPVL2111 contained the full length or almost full length of the objective nucleotide sequence encoding PVL.

### 〈3〉 Confirmation of PVL clone sequence

### 1. Nucleotide sequence determination of PVL cDNA

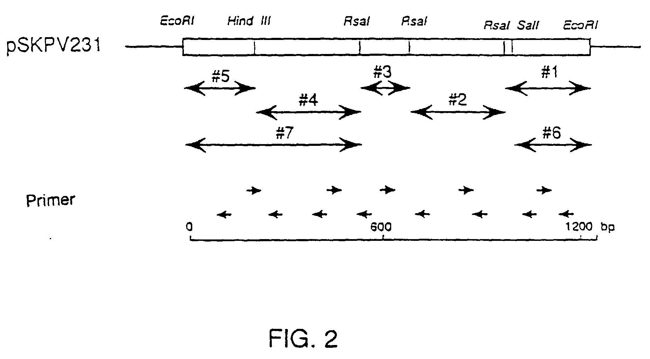

The inserts were excised from λPVL231 and λPVL2111 by a treatment with *Eco*RI, and inserted into the *Eco*RI site of pBluescript SK(+) (Stratagene) to obtain SKPVL231 and SKPVL2111, respectively. When they were digested with various restriction enzymes, and analyzed by electrophoresis, the inserts of these two clones exhibited the same pattern (data not shown). Therefore, it was decided to mainly analyze SKPVL231. The determination of nucleotide sequence was performed by using ABI Prism Primer Cycle Sequencing reaction Kit (Perkin-Elmer Co.). As a thermal cycler, Geneamp PCR system 9600 (Perkin-Elmer Co.) was used. As a sequencer, Model 373A (Perkin-Elmer Co.) was used. The determined nucleotide sequence was analyzed by using Wisconsin DNA analysis program (The Wisconsin Sequence Analysis Package™). As shown in Figure 2, seven fragments obtained by digesting the insert with *Rsa*I, *Hind*III, and *Sal*I were inserted into pBluescript SK(+) to form seven subclones, and sequencing was performed several times from the both directions for each of the seven clones by using M13-20 primer (Promega) and M13 reverse primer (Promega) to determine an insert nucleotide sequence of each subclone. Further, in order to establish the entire nucleotide sequence of the insert contained in SKPVL231, 13 primers shown in Figure 2 were prepared based on the obtained information, and the nucleotide sequence of the whole insert was determined by utilizing them.

### 2. Analysis of nucleotide sequence of PVL cDNA

The results of the nucleotide sequence analysis are shown in Figure 3. The full length of the insert DNA was 1256 bp, and did not contain poly A tail and related signals. This is considered to be caused by the use of random primers for the library preparation. When the provided insert DNA was searched for ORF, only one ORF not shorter than 900 bp was confirmed, and only ORFs of 500 bp or shorter were observed in four frames among the six frames. As for the remaining one frame, neither initiation codon nor stop codon was observed. While this may be considered a part of larger ORF, it is unlikely to encode the desired substance because the sequence obtained from the amino acid sequence analysis was not contained in this frame at all, and because a longer ORF containing the sequence obtained from the amino acid sequence analysis is unlikely to be present since 1256 bp by themselves correspond to a molecular weight of more than 43 kDa when converted into amino acids. The ORF considered to encode PVL consisted of 1206 bp in the number of nucleotide, which corresponded to 402 amino acid residues. The amino acid sequence expected from the nucleotide sequence contained all of the partial amino acid sequences of the four peptides which were obtained from the amino acid sequence analysis of the fragments resulted from the enzymatic digestion of PVL (underlined with bold lines in Figure 3). Further, this ORF contains five ATG sequences other than the first ATG sequence, but if these were considered as initiation codons, sequences not containing the sequences obtained in the amino acid sequence analysis would exist even for the longest ORF among them, and it did not agree with the results. The molecular weight expected from the nucleotide sequence of the ORF considered to encode PVL was 43006.88, and was larger than the molecular weight estimated by SDS-PAGE or gel filtration (40 kDa). Furthermore, the amino acid composition expected from the nucleotide sequence of this ORF generally agreed well in amino acid ratios with the results of the previously reported amino acid composition analysis of PVL (N. Kochibe et al., J. Biol. Chem., 264, 173-177 (1989))(Table 1). In Table 1, the numerals represent numbers of amino acid residues, and parenthesized numerals represent amino acid ratios (%).

**Table 1**

| Comparison of amino acid composition | | |
|---|---|---|
| Amino acid | Amino acid composition expected from cDNA nucleotide sequence (43 kDa) | Amino acid composition of PVL reported by N. Kochibe et al. (40 kDa) |
| Asx | 60 (14.9) | 49.8 (13.7) |
| Thr | 22 (5.5) | 27.7 (7.6) |
| Ser | 13 (3.2) | 17.7 (4.9) |
| Glx | 17 (4.2) | 24.5 (6.7) |
| Pro | 13 (3.2) | 15.6 (4.3) |
| Gly | 60 (14.9) | 42.3 (11.6) |
| Ala | 32 (8.0) | 27.7 (7.6) |
| Cys | 4 (1.0) | 2.5 (0.7) |
| Val | 42 (10.4) | 23.1 (6.3) |
| Met | 6 (1.5) | 3.5 (1.0) |
| Ile | 21 (5.2) | 18.5 (5.1) |
| Leu | 28 (7.0) | 27.0 (7.4) |
| Tyr | 10 (2.5) | 10.7 (2.9) |
| Phe | 22 (5.5) | 23.5 (6.5) |
| His | 7 (1.7) | 7.8 (2.1) |
| Lys | 15 (3.7) | 16.4 (4.5) |
| Trp | 9 (2.2) | 1.5 (0.4) |
| Arg | 21 (5.2) | 24.5 (6.7) |
| Total | 402 | 364.3 |

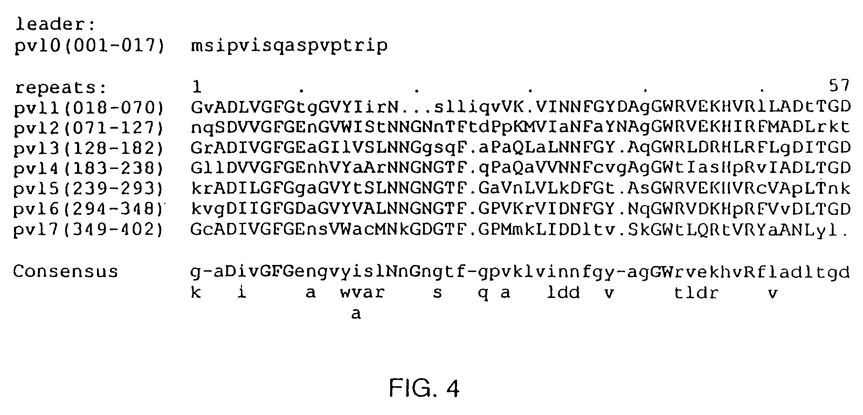

The values of the amino acid composition analysis of PVL reported by N. Kochibe were numbers of amino acid residues calculated from the molecular weight which was considered 40 kDa and each amino acid ratio. Therefore, the amino acids having a large ratio in the composition apparently show a large difference compared with the number of amino acid residue obtained from the nucleotide sequence of the cDNA. However, the ratios show good agreement. As for tryptophan, the difference of the values between the both results seems to be relatively large. This may be considered to be caused by decomposition of tryptophan itself due to hydrolysis treatment of peptides with an acid during the amino acid composition analysis. As underlined with normal lines in Figure 3, there were five sites which can be N-glycosylated. Interestingly, a repetition sequence iterated seven times over the entire protein was observed as shown in Figure 4. While a repeated structure in amino acid sequence has been observed in other plant lectins, they were only a locational repetition, and it is extremely unusual that repetition sequences constitute 90% or more of the protein.

GenBank, EMBL and a protein data base (PIR, SWISS-PROT) were searched for all species, not only fungi, but any species exhibiting significant homology were not retrieved.

### 3. Expression of PVL cDNA

### 3-1. Expression utilizing E. coli as host cell

### 3-1-1. Expression utilizing expression vector PinPointXa

The fragment obtained by digesting SKPVL231 with *Hind*III was inserted into the *Hind*III site of expression vector Pinpoint Xa-1 (Promega) to construct pPPVL231. *E. coli* strain JM109 was transformed with this plasmid. This transformed *E. coli* strain was precultured overnight in LB containing 50 µg/ml ampicillin (LBamp). The precultivation broth (1 ml) was added to 100 ml of LBamp, cultured at 37°C for one hour with shaking, and IPTG was added thereto to a final concentration of 100 µM, and it was further cultured for four hours with shaking. 100 µl of the culture medium was taken and centrifuged at 10,000 x g at room temperature for five minutes, and the supernatant was discarded. To the pellet, a buffer (sample buffer) containing 50 mM Tris-HCl, 2% SDS, 20% glycerol, 5% β-mercaptoethanol, and 0.002% BPB was added, and the mixture was stirred, and boiled at 95°C for five minutes. Then, the mixture was analyzed by SDS-PAGE and Western blotting.

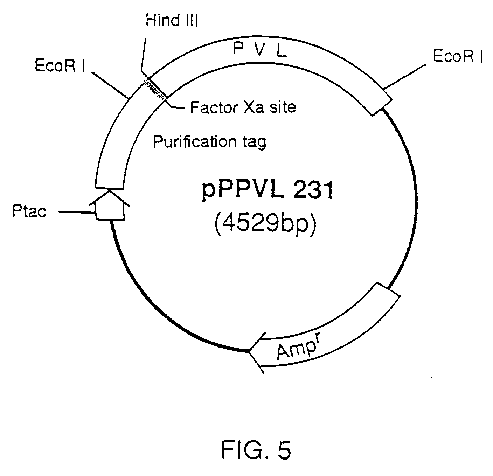

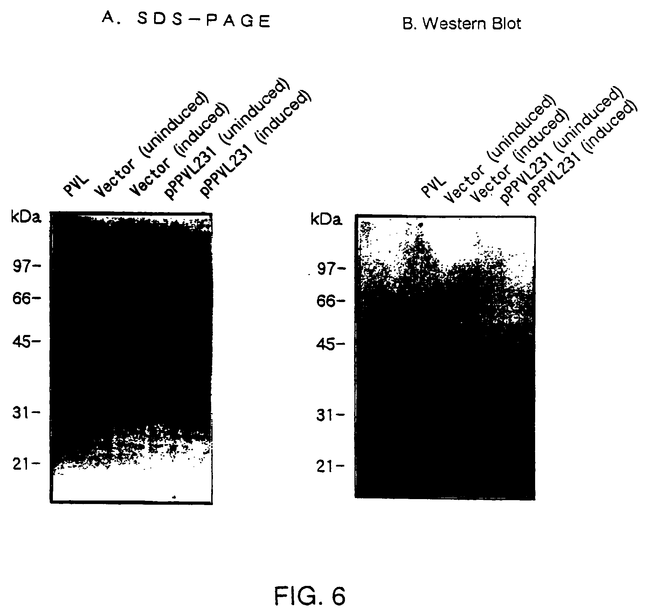

The structure of the expression vector pPPVL231 is shown in Figure 5. The expression vector Pinpoint Xa-1 expresses a fusion protein of a peptide which is biotinylated in *E. coli*. The insert, which was inserted at the *Hind*III site in the downstream of the biotinylated peptide gene of this vector, lacked first 23 bp of the ORF of PVL cDNA, that corresponded to 8 amino acids. In the analysis by SDS-PAGE (Fig. 6.A), a band of 53 kDa was identified in the extract of *E. coli* transformed with pPPVL231, which was not observed in the extract of *E. coli* transformed only with the vector (Pinpoint Xa-1). Further, in the analysis by Western blotting (figure 6.B), the same 53 kDa band specifically reacted with the anti-PVL antibody, and any band reactive with the anti-PVL antibody was not identified in the extract of *E. coli* introduced only with the vector (Pinpoint Xa-1).

Though data were not shown, the molecular weight of PVL expressed in a form which was not a fusion protein was confirmed by SDS-PAGE to be 40 kDa, which was the same as that of naturally-occurring PVL.

After purification by using the avidin resin (Softlink SoftRelease Avidin Resin, Promega) contained in Pinpoint protein purification system (kit available from Promega) and according to the known protocol attached to the kit, the recombinant fusion protein exhibited a lectin activity and an agalacto-IgG-binding property as determined by the methods mentioned above.

From these results, it was demonstrated that the cloned cDNA encoded PVL.

### 3-1-2. Expression utilizing expression vector pGEMEX

A *Sac*I-*Eco*RI-cleaved DNA fragment of pPPVL231 containing the PVL gene was inserted into a corresponding *Sac*I-*Eco*RI site of plasmid pGEMEX-1 (Promega) to form a plasmid pGEMEXPVL231.

*E. coli* BL21(DE3)pLysS was transformed with the pGEMEXPVL231. *E. coli* transformed with the plasmid was cultured overnight on an LB-ampicillin plate (50 µg/ml), and a resulting single colony was inoculated into LB-ampicillin (50 µg/ml) liquid culture medium, and precultured at 37°C overnight. To LB culture medium, a 1/100 volume of the preculture broth was inoculated, cultured at 37°C for 1 hour with shaking, and IPTG was added thereto to a final concentration of 100 µM. After further cultivation at 37°C for 4 hours with shaking, the cells were collected by centrifugation. To the cell pellet, Solution A (50 mM Tris-HCl (pH 8.0), 145 mM NaCl, 5 mM EDTA, 0.5 mM PMSF (phenylmethanesulfonyl fluoride), 10% glycerol) was added in an amount of 10 times of the wet weight of the pellet, and the mixture was sonicated. This sonicated bacterial cell mixture was centrifuged at 20,000 x g for 30 minutes at 4°C, and the supernatant was taken as a crude cell extract.

The extract was analyzed by SDS-PAGE and Western blotting, and as a result, 40 kDa and 56 kDa bands which specifically reacted with the anti-PVL antibody were detected. From these results, the expression of recombinant PVL from pGEMEXPVL231 was confirmed.

The product from pGEMEXPVL231 was a fusion protein of PVL and the gene10 product of T7 phage, and the molecular weight of this product well agreed with the molecular weight expected from the sequence.

### 3-1-3. Expression utilizing expression vector pET-5

A *Hind*III-EcoRI-cleaved DNA fragment of pSKPVL231 containing the PVL gene was inserted into a corresponding *Hind*III-*Eco*RI site of plasmid pET-5c (Promega) to form a plasmid pETPVL231.

*E. coli* BL21(DE3)pLysS was transformed with the pETPVL231, and a crude cell extract was obtained in the same manner as in the above 3-1-2.

The extract was analyzed by SDS-PAGE and Western blotting, and as a result, 40 kDa and 56 kDa bands which specifically reacted with the anti-PVL antibody were detected. From these results, the expression of recombinant PVL from pETPVL231 was confirmed.

The product from pETPVL231 contained the same sequence as the gene 10 product of T7 phage at its amino terminus, and the molecular weight of this product approximately corresponded to that of naturally-occurring PVL as expected from the sequence.

### 3-1-4. Expression utilizing expression vector pGEX-5X

A restriction enzyme BamHI-*Eco*RI-cleaved DNA fragment of pETPVL231 containing the PVL gene, which plasmid was prepared in the above 3-1-2., was inserted into a corresponding BamHI-EcoRI site of a plasmid pGEX-5X-1 (Pharmacia) to form a plasmid pGSTPVL231.

*E. coli* ABLE K was transformed with the pGSTPVL231, and a crude cell extract was obtained in the same manner as in the above 3-1-2.

The extract was analyzed by SDS-PAGE and Western blotting, and as a result, a specific 68 kDa band was identified. This band was specifically reacted with the anti-PVL antibody in the Western blotting.

From these results, the expression of recombinant PVL from pETPVL231 was confirmed.

The product from pGSTPVL231 was a fusion protein of glutathione-S-transferase and PVL, and the molecular weight of this product was well agreed with the molecular weight expected from the sequence.

### 3-2. Expression utilizing yeast as host cell

### 3-2-1. Construction of yeast expression plasmid

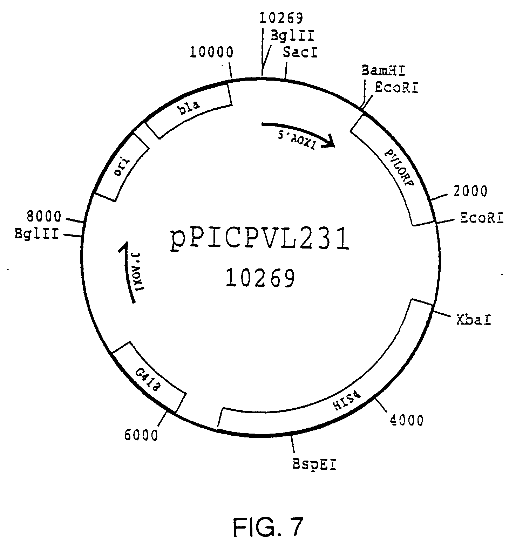

The plasmid pSKPVL231 was digested with restriction enzyme *Dde*I, blunt-ended with DNA polymerase Klenow fragment, and ligated with an *Eco*RI-linker. This reaction product was digested with restriction enzyme *Eco*RI, and subjected to agarose gel electrophoresis to separate and purify a DNA fragment containing the PVL gene, and a plasmid pPICPVL231 composed of yeast expression plasmid pPIC3K (Invitrogen) in which the PVL gene sequence was inserted into its *Eco*RI cleavage site in such a direction that the PVL gene should be transcribed in the downstream of *AOX1* (alcohol oxidase) gene promoter. The structure of the expression plasmid pPICPVL231 is shown in Figure 7. This plasmid contained the entire sequence of the protein-encoding region of the PVL gene cDNA.

This expression plasmid (pPICPVL231) contains a replication origin for replication in *E. coli* (ori; pMB1 replication origin), and an ampicillin resistance gene (bla), and can multiply in *E. coli*. 5'AOX1 is a promoter region of the alcohol oxidase gene *AOX1*, and the ORF (open reading frame) of the PVL gene is inserted in the downstream of *AOX1*. While this plasmid does not contain a replication origin region for replication in yeast (ARS), it can be replicated as a part of chromosome when it is inserted into a chromosome.

For example, when this plasmid is digested with *Bsp*E1, and introduced into yeast by means of electroporation or the like, it is integrated into the HIS4 region. Similarly, when it is digested with *Sac*I, it is incorporated into the *AOX1* gene. Gene replacement with the *AOX1* gene is also possible by introducing the plasmid digested with *Bg*lII.

### 3-2-2. Introduction of expression plasmid and selection of clone

The plasmid DNA of pPICPVL231 was digested with a restriction enzyme *Bsp*EI, and introduced into yeast *Pichia* pastoris, SMD1168 (*his*4 *pep*4) by electroporation. Gene transductants that could grow without histidine were selected on MD agar medium (Minimal Dextrose Medium, 1.35% Yeast Nitrogen Base, 4 x 10⁻⁵% biotin, 2% glucose, 2% agarose). The transductants had pPICPVL231 DNA inserted into the *his*4 gene region as a part of yeast chromosome of *Pichia pastoris*. Among the transductants, a highly G418-resistant clone (designated as #20) was selected expecting an increase of an expression level due to the gene duplication.

### 3-2-3. Expression of PVL

A single colony of the aforementioned clone #20 was cultured in 10 ml of MG (Minimal glycerol medium; 1.34% Yeast Nitrogen Base, 4 x 10⁻⁵% biotin, 1% glycerol) at 30°C overnight. After the cells were collected, they were cultured at 37°C in 30 ml of MM (Minimal methanol medium; 1.34% Yeast Nitrogen Base, 4 x 10⁻⁵% biotin, 1.5% methanol), to which methanol was added in an amount corresponding to 0.5% of the culture medium every 24 hours.

Each 1-ml sample was taken at 24 hour-intervals, and centrifuged, and the pellet was stored at -80°C. Each sample was suspended in 100 µl of BB (Breaking Buffer; 50 mM sodium phosphate, pH 7.4, 1 mM EDTA, 5% glycerol), and cell walls were broken with glass beads, and centrifuged at 12,000 rpm for ten minutes. To the supernatant resulting from the centrifugation, an SDS sample buffer was added, and the mixture was subjected to SDS-PAGE, and analyzed by Western blotting. As a result, expression of a protein having a molecular weight substantially equivalent to that of the naturally-occurring PVL and reactive with the anti-PVL antibody was observed in all of the samples.

Based on the fact that expression of the PVL gene in yeast with this expression plasmid (pPICPVL231) was confirmed, it can readily be predicted by those skilled in the art that the expression of PVL would be possible by a similar *Pichia pastoris* expression vector utilizing an *AOX1* gene promoter, such as pPIC9K, pAO815, and pHIL-D2. It is also expected that expression of the PVL gene would be possible by an expression system of various yeasts including *Pichia pastoris*, *Saccharomyces cerevisiae* and *Schizosaccharomyces pombe*. Further, it is expected that improvement of these expression plasmids including pPICPVL231, selection and improvement of transductants, and improvement of cultivation and induction conditions may lead to large scale expression and supply of recombinant PVL.

### Preparation Example 1 (Preparation of agalacto-IgG)

5 mg of human IgG, 30 mU of streptococal β-galactosidase, and 0.21 mU of sialidase were dissolved in 1 ml of 100 mM citric acid buffer, pH 6.5. This mixed solution was allowed to react at 37°C for 18 hours, and then the reaction was terminated by a heat treatment at 56°C for ten minutes. This solution was adjusted to pH 8.9 to 9.2 by adding 5 to 10 times volume of Protein A cellulofine (Seikagaku Corporation) binding buffer (3 M NaCl, 1.5 M glycine, pH 9.0). Then, to the solution, 2 g of Protein A cellulofine was added, and the solution was gently stirred at room temperature for 10 minutes. The resulting suspension was centrifuged at 1000 rpm for one minute to collect the Protein A cellulofine. To the collected Protein A cellulofine, 1 to 2 ml of Protein A cellulofine elution buffer (0.1 M citric acid buffer, pH 5) was added, and the mixture was left at room temperature for five minutes. Then, it was centrifuged at 1000 rpm for one minute, and 0.2 ml of 2 M Tris was added to the collected supernatant. This solution was dialyzed against phosphate buffered saline (pH 7.2 to 7.5, free of divalent ions, referred to as PBS(-) hereinafter), and the resulting solution was used as an agalacto-IgG standard.

### Comparative Example 1: Quantitative assay of agalacto-IgG by known assay method

The agalacto-IgG was assayed by using a known method for directly assaying the agalacto-IgG (J. Immunol., 151, 1137-1146 (1993)). That is, a commercially available protein G was diluted to 20 µg/ml in 0.1 M carbonate buffer (pH 8.5 to 9), and 50 µl (1 µg/well) of this solution was added to each well of a Nunc immunoplate (trade name; Maxisorp, Nunc), and left at 4°C for 16 hours to uniformly coat each well.

This plate was washed twice with PBS(-), and PBS(-) solution containing 3% bovine serum albumin (BSA, sold by Seikagaku Corporation) was added as a blocking agent, and left stand at room temperature for two hours to coat the portions of the wells on which protein G was not immobilized.

Then, the plate was washed three times with a washing solution (PBS(-) containing 0.05% Tween 20, Wako Pure chemicals Industries), and 50 µl each of standard solutions at various concentrations (1.25 to 20 µg/ml) of the agalacto-IgG purified in Preparation Example 1 was added, and left stand at 37°C for 60 minutes for reaction. As the solvent of the agalacto-IgG standard solutions, PBS(-) containing 1% BSA and 0.05% Tween 20 (referred to as "reaction diluent" hereinafter) was used.

This plate was washed three times with the washing solution, and 25 µl of 40 µg/ml biotin-labeled PVL diluted with the reaction diluent and 25 µl of streptavidin-labeled peroxidase (Seikagaku Corporation) diluted 1250 times with the reaction diluent were added, and left stand at 37°C for 60 minutes for reaction. Further, the plate was washed three times with the washing solution, and 50 µl of tetramethylbenzidine solution (Moss Inc., abbreviated as TMB hereinafter) was added as a substrate for the peroxidase, and allowed to react at 37°C for 15 minutes for color development.

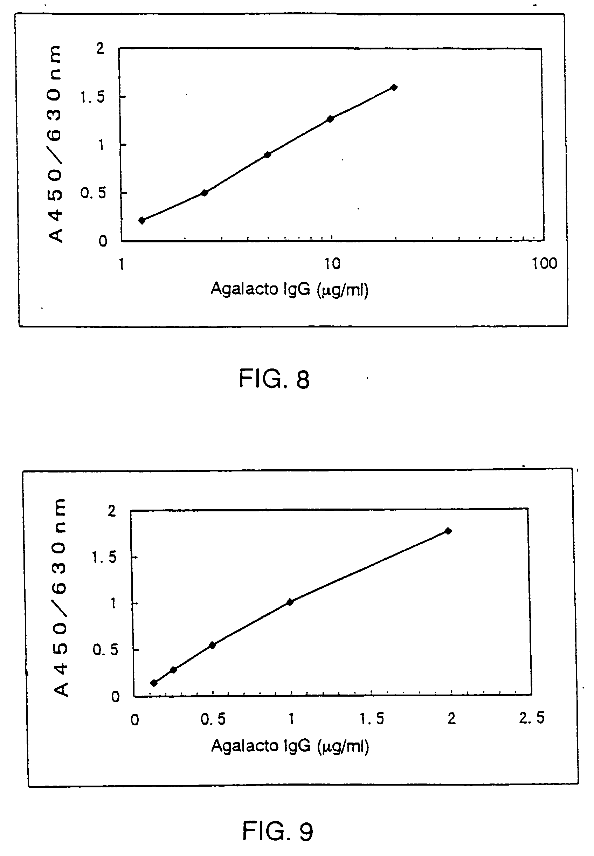

After the color development, 50 µl of 1 N HCl was added to the plate to terminate the reaction, and absorbance at a wavelength of 450 nm of the solution colored by the decomposition of TMB was measured (reference wavelength: 630 nm, A450/630) by Well Reader SK601 (sold by Seikagaku Corporation). The obtained results are shown in Figure 8.

From the results, it can be seen that 1.25 to 20 µg/ml of agalacto-IgG can be guantitated by this assay method.

### Example 3: Quantitative assay of agalacto-IgG

PVL purified in Example 1 or commercially available PVL was diluted to 20 µg/ml in 0.1 M carbonate buffer containing 10% glycerol (pH 8.5 to 9), and 50 µl of this solution (1 µg/well) was added to each well of a Nunc immunoplate (trade name; Maxisorp, Nunc), and left at 4°C for 16 hours to uniformly coat each well.

This plate was washed twice with PBS(-), and PBS(-) solution containing 3% bovine serum albumin (BSA, sold by Seikagaku Corporation) was added as a blocking agent, and left stand at room temperature for two hours to coat the portions of the wells on which PVL was not immobilized.

Then, the plate was washed three times with a washing solution (PBS(-) containing 0.05% Tween 20), and 50 µl each of standard solutions at various concentrations (0.125 to 2 µg/ml) of the agalacto-IgG purified in Preparation Example 1 was added, and left stand at 37°C for 60 minutes for reaction. As the solvent of the agalacto-IgG standard solutions, PBS(-) containing 1% BSA and 0.05% Tween 20 (referred to as "reaction diluent" hereinafter) was used.

Then, this plate was washed three times with the washing solution, and 50 µl of 0.3 µg/ml peroxidase-labeled anti-human IgG antibody (sold by Seikagaku Corporation) diluted with the reaction diluent was added, and left stand at 37°C for 60 minutes for reaction. Further, the plate was washed three times with the washing solution, and 50 µl of TMB solution was added as a substrate for the peroxidase, and allowed to react at 37°C for 15 minutes for color development.

After the color development, 50 µl of 1 N HCl was added to the plate to terminate the reaction, and absorbance at a wavelength of 450 nm of the solution colored by the decomposition of TMB was measured (reference wavelength: 630 nm, A450/630) by Well Reader SK601 (sold by Seikagaku Corporation). The obtained results are shown in Figure 9.

From the results, it can be seen that 0.125 to 2 µg/ml of agalacto-IgG can be quantitated by the assay method of the present invention. This assay method is 10 times more sensitive compared with the assay method mentioned in Comparative Example 1, and does not require expensive protein G, and biotin-labeled PVL, which is difficult to be prepared due to the agglutination property of PVL itself. Thus, it was demonstrated that the assay method of the present invention was a sensitive and simple method for assaying the agalacto-IgG.

### Example 4: Influence of various monosaccharides on assay system

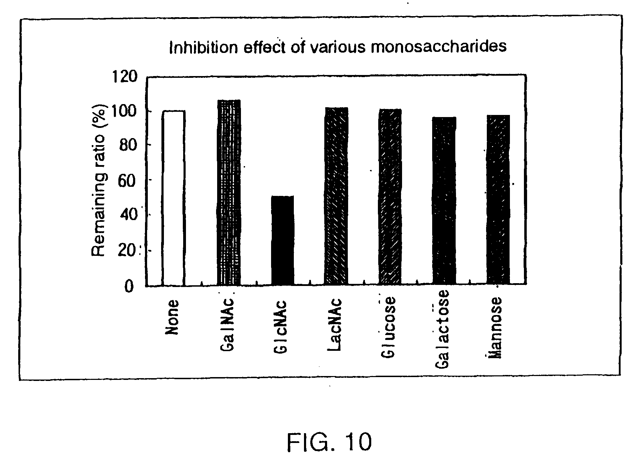

To an agalacto-IgG standard solution (2.5 µg/ml), N-acetylgalactosamine (GalNAc), N-acetylglucosamine (GlcNAc), N-acetyllactosamine (LacNAc), glucose, galactose, or mannose was added to a final concentration of 50 mM, and an assay was performed according to the assay method of Example 3 to examine the effect of the various monosaccharides on the assay system. The results are shown in Figure 10. The results are represented in percentage (%) as a remaining ratio, which is a ratio of a value obtained in the presence of each monosaccharide relative to the value obtained without the addition of monosaccharide which is defined as 100%. From the results of Figure 10, it is clear that about 50% inhibition was observed in the presence of 50 mM of GlcNAc. The other monosaccharides showed no influence at all, and this indicates that the assay method of the present invention is an assay method relying on the binding GlcNAc at the complex type binary sugar chain terminus present in the agalacto-IgG molecule, i.e., a method capable of directly and specifically assaying the agalacto-IgG.

### Example 5: Quantitative assay of agalacto-IgG in sera of normal subjects, and patients of knee osteoarthritis, rheumatoid arthritis and liver disease

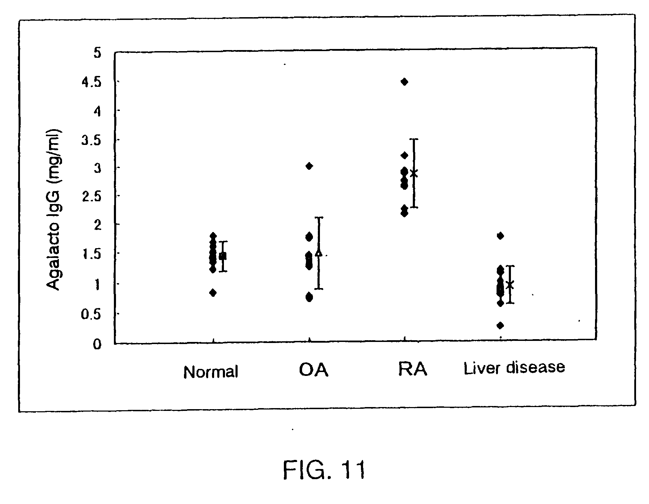

An assay of the agalacto-IgG present in sera of normal subjects (10 subjects), knee osteoarthritis patients (abbreviated as "OA" hereinafter, 10 cases), rheumatoid arthritis (abbreviated as "RA" hereinafter) patients (10 cases), and liver disease patients (15 cases) was performed according to the method described in Example 3. The obtained assay results are shown in Table 2 and Figure 11. As seen from the results shown in Table 2 and Figure 11, the average serum agalacto-IgG of RA patients was about two times as high as that of the normal subjects. The serum agalacto-IgG of the OA patients was substantially equivalent to that of the normal subjects. The serum agalacto-IgG of the liver disease patients was slightly lower than the serum agalacto-IgG of the normal subjects. From these results, it was demonstrated that assaying serum agalacto-IgG enables differential diagnosis of RA specifically distinguishing RA from the other diseases.

**Table 2**

| Serum agalacto-IgG content of normal subjects, OA patients, RA patients and liver disease patients | | |
|---|---|---|
| Sample | Average (mg/ml) | ± S.D. |
| Normal subjects | 1.44 | 0.25 (n=10) |
| OA patients | 1.48 | 0.60 (n=10) |
| RA patients | 2.85 | 0.61 (n=10) |
| Liver disease patients | 0.93 | 0.31 (n=15) |

### Example 6: Agalacto-IgG assay kit of the present invention

An agalacto-IgG assay kit was constituted from the following components:

| | |
|---|---|
| 1. Lectin-immobilized plate | 1 plate |

| 2. Agalacto-IgG standard solutions | |
|---|---|
| 10 µg/ml 0.5 ml lyophilized product | 1 ampoule |
| 5 µg/ml 0.5 ml lyophilized product | 1 ampoule |
| 2.5 µg/ml 0.5 ml lyophilized product | 1 ampoule |
| 1.25 µg/ml 0.5 ml lyophilized product | 1 ampoule |
| 0.6 µg/ml 0.5 ml lyophilized product | 1 ampoule |
| 0.3 µg/ml 0.5 ml lyophilized product | 1 ampoule |
| The agalacto-IgG standard solutions were prepared with a mixture of 1% BSA (dissolved in PBS), 0.05% Tween 20, and 0.05% Proclin (sold by Iwaki, Supelco Inc.), and then lyophilized. | |
| 3. Horseradish peroxidase-labeled anti-human IgG antibody solution 10 ml | 1 ampoule |
| 4. TMB solution 10 ml | 1 ampoule |
| 5. Reaction termination solution (1 N HCl) 10 ml | 1 ampoule |
| 6. Sample diluent stock solution (used by diluting 5 times) 40 ml | 1 ampoule |
| This sample diluent stock solution is composed of a mixture of 5% BSA (dissolved in PBS of 5-fold concentration), 0.25% Tween 20 and 0.25% Proclin. | |
| 7. Washing stock solution (used by diluting 5 times) 40 ml | 1 ampoule |
| This washing stock solution is composed of PBS(-) of 5-fold concentration containing 0.25% Tween 20. | |
| 8. Control standard solution (serum) 50 µl | 1 ampoule |

### Industrial Applicability

The method for assaying the agalacto-IgG of the present invention enables direct and specific measurement of the agalacto-IgG in a sample such as serum with extremely high sensitivity, at low cost and with simple procedure, and can eliminate the problems concerning the labeling of the lectin and instability of the lectin. The detection method based on this assay method is an effective method as a specific detection method capable of accurately distinguishing OA and RA in the diagnosis of arthritis. The kits of the present invention are also extremely useful as kits exhibiting similar advantages.

The assay method of the present invention utilizing an anti-experimental animal IgG antibody enables an assay of an agalacto-IgG in an RA model animal (e.g., a model animal where RA is induced by an chemical agent, a model animal naturally suffering RA etc.), and can be a useful evaluation method for elucidation of the cause of RA crisis and development of pharmaceutical drugs.

Further, the present invention also provides a DNA encoding PVL or an equivalent thereof, and polypeptides expressed by the DNA or DNA fragments derived therefrom.

Since the DNA encoding PVL or the equivalent thereof is obtained by the present invention, it is expected that PVL or the equivalent thereof can be prepared in such a large scale that its industrial use can be realized. It is expected that it is utilized for applications for which PVL has conventionally been used, or applications utilizing the binding specificity or the like of PVL.

Furthermore, the recombinant PVL obtained by the present invention is expected to be applied to diagnostic agents. That is, there has been reported abnormal increase of IgG which lacks a part containing galactose (Gal) of the sugar chain binding to an asparagine residue at position 297, and has exposed basic structure of N-binding type sugar chain, Asn←GlcNAc←GlcNAc←Man←GlcNAc (agalacto-IgG) in rheumatoid arthritis (RA) patient serum, unlike normal subjects (Mullinax, F., Arthritis Rheum., 18, 417 (1975); Parekh, B. et al., Nature, 316, 452 (1985)). On the other hand, PVL is a monomer protein, and is bound to a sugar chain having GlcNAc at a terminus such as βGlcNAc1→6Man and 1 →3Man (Kochibe, A. and Matta, K., J. Biol. Chem., 264, 173-177 (1989)). Further, possibility of application of PVL to diagnostic methods of RA has also been reported, since PVL specifically recognizes the agalacto-IgG (Tsuchiya, N. and Kobata, A., J. Immunol., 151, 1137-1146 (1993)). Moreover, the specific increase of the agalacto-IgG has also been reported for some other diseases. Therefore, the application of the recombinant PVL to diagnostic agents for such diseases is strongly expected. Further, it is also expected that lectins having novel activities could be created by genetic engineering techniques utilizing the DNA of the present invention.

## Claims

1. A method for assaying an agalacto-IgG in a sample, which comprises a step of reacting the agalacto-IgG in the sample with a lectin immobilized on a solid phase to form a complex of the agalacto-IgG and the lectin immobilized on the solid phase.

2. The method according to claim 1, wherein the complex of the agalacto-IgG and the lectin immobilized on the solid phase is detected with an anti-IgG antibody.

3. The method according to claim 2, which comprises the step of reacting the agalacto-IgG in the sample with the lectin immobilized on the solid phase to form the complex of the agalacto-IgG and the lectin immobilized on the solid phase, and a step of reacting the agalacto-IgG with the anti-IgG antibody.

4. A method for assaying an agalacto-IgG in a sample, which comprises a step of sandwiching the agalacto-IgG between an lectin and an anti-IgG antibody.

5. The method according to any one of claims 1 to 4, wherein the lectin is a lectin which specifically binds to a β-N-acetylglucosamine residue.

6. The method according to any one of claims 1 to 4, wherein the lectin is a *Psathyrella velutina* lectin.

7. The method according to any one of claims 2 to 6, wherein the anti-IgG antibody is an anti-IgG antibody labeled with a labeling substance, or an anti-IgG antibody which can be labeled with a labeling substance.

8. The method according to any one of claims 2 to 7, wherein the anti-IgG antibody is an anti-human IgG antibody.

9. A method for detecting rheumatism, which comprises assaying an agalacto-IgG by utilizing the method for assaying the agalacto-IgG as defined in any one of claims 1 to 8.

10. The method according to claim 9, wherein rheumatism is rheumatoid arthritis.

11. A kit for assaying agalacto-IgG, which is used in a method for assaying an agalacto-IgG by forming a complex of the agalacto-IgG and a lectin, and comprises the lectin immobilized on a solid phase as a component.

12. A kit for diagnosing rheumatism, which is used in a method for diagnosing rheumatism based on an assay of an agalacto-IgG by forming a complex of the agalacto-IgG and a lectin, and comprises the lectin immobilized on a solid phase as a component.

13. A polypeptide of lectin comprising at least an amino acid sequence represented by the following formula (1):
Asp Xaa Xaa Gly Phe Gly Xaa Xaa Xaa Xaa Xaa Xaa Xaa Xaa Asn (Xaa) (Gly) (Xaa) Xaa Xaa Xaa (Xaa) Xaa Xaa Xaa Xaa (Xaa) Xaa Xaa Xaa Xaa Xaa Xaa Xaa (Xaa) Xaa Xaa Gly Trp Xaa Xaa Xaa Xaa Xaa Xaa Arg [Formula (1)]
wherein each Xaa is an arbitrary amino acid, and each parenthesized amino acid may be absent.

14. A polypeptide of lectin comprising at least a sequence corresponding to one cycle starting from an arbitrary position of a cyclic amino acid sequence represented by the following formula (2):
Xaa Xaa Xaa Asp Xaa Xaa Gly Phe Gly Xaa Xaa Xaa Xaa Xaa Xaa Xaa Xaa Asn (Xaa) (Gly) (Xaa) Xaa Xaa Xaa (Xaa) Xaa Xaa Xaa Xaa (Xaa) Xaa Xaa Xaa Xaa Xaa Xaa Xaa (Xaa) Xaa Xaa Gly Trp Xaa Xaa Xaa Xaa Xaa Xaa Arg Xaa Xaa Xaa Xaa Xaa Xaa Xaa (Xaa) [Formula (2)]
Wherein the sequence of the above formula is a cyclic sequence, and wherein each Xaa is an arbitrary amino acid, and each parenthesized amino acid may be absent.

15. The polypeptide of lectin according to claim 13, which comprises at least an amino acid sequence represented by the following formula (3):
Asp Xaa1 Xaa1 Gly Phe Gly Xaa Xaa Xaa Xaa1 Xaa Xaa Xaa Xaa Asn (Xaa) (Gly) (Xaa) Xaa Xaa Xaa1 (Xaa) Xaa Xaa1 Xaa1 Xaa (Xaa) Xaa1 Xaa1 Xaa Xaa Xaa1 Xaa Xaa (Xaa) Xaa Xaa Gly Trp Xaa Xaa1 Xaa Xaa Xaa Xaa1 Arg Xaa Xaa1 Xaa1 [Formula (3)]
wherein each Xaa is an arbitrary amino acid, each Xaa1 is a hydrophobic amino acid, and parenthesized amino acid may be absent.

16. The polypeptide of lectin according to any one of claims 13 to 15, which comprises at least an amino acid sequence represented by the following formula (4):
Xaa Xaa Xaa Asp Xaa1 Xaa1 Gly Phe Gly Xaa Xaa Xaa Xaa1 Xaa Xaa Xaa Xaa Asn (Xaa) (Gly) (Xaa) Xaa Xaa Xaa1 (Xaa) Xaa Xaa1 Xaa1 Xaa (Xaa) Xaa1 Xaa1 Xaa Xaa Xaa1 Xaa Xaa (Xaa) Xaa Xaa Gly Trp Xaa Xaa1 Xaa Xaa Xaa Xaa1 Arg Xaa Xaa1 Xaa1 Xaa Xaa Xaa Xaa (Xaa) [Formula (4)]
wherein each Xaa is an arbitrary amino acid, each Xaa1 is a hydrophobic amino acid, and each parenthesized amino acid may be absent.

17. The polypeptide of lectin according to any one of claims 13 to 16, which comprises at least an amino acid sequence represented by the following formula (5):
Asp X11 X11 Gly Phe Gly Xaa Xaa Xaa X12 Xaa Xaa Xaa Xaa Asn (Xaa) (Gly) (Xaa) Xaa Xaa X13 (Xaa) Xaa X14 X15 Xaa (Xaa) X16 X11 Xaa Xaa X13 Xaa Xaa (Xaa) Xaa Xaa Gly Trp Xaa X11 Xaa Xaa Xaa X17 Arg Xaa X18 X19 [Formula (5)]
wherein each Xaa is an arbitrary amino acid, each X11 is Ile, Val or Leu, X12 is Ile or Val, each X13 is Phe or Leu, X14 is Pro, Ala, or Val, X15 is Pro, Ala, Val, or Met, X16 is Ala, Val or Leu, X17 is Pro, Val, Leu or Ile, X18 is Ala, Val, Leu, Ile or Met, and X19 is Ala, Val or Gly, and each parenthesized amino acid may be absent.

18. The polypeptide of lectin according to claim 17, which comprises at least an amino acid sequence represented by the following formula (6):
Xaa Xaa Xaa Asp X11 X11 Gly Phe Gly Xaa Xaa Xaa X12 Xaa Xaa Xaa Xaa Asn (Xaa) (Gly) (Xaa) Xaa Xaa X13 (Xaa) Xaa X14 X15 Xaa (Xaa) X16 X11 Xaa Xaa X13 Xaa Xaa (Xaa) Xaa Xaa Gly Trp Xaa X11 Xaa Xaa Xaa X17 Arg Xaa X18 X19 Xaa Xaa Xaa Xaa (Xaa) [Formula (6)]
wherein each Xaa is an arbitrary amino acid, each X11 is Ile, Val or Leu, X12 is Ile or Val, each X13 is Phe or Leu, X14 is Pro, Ala, or Val, X15 is Pro, Ala, Val, or Met, X16 is Ala, Val or Leu, X17 is Pro, Val, Leu or Ile, X18 is Ala, Val, Leu, Ile or Met, and X19 is Ala, Val or Gly, and each parenthesized amino acid may be absent.

19. The polypeptide of lectin according to any one of claims 13 to 18, which comprises one or more amino acid sequences selected from the amino acid sequences shown in SEQ ID NOS: 3 to 9.

20. The polypeptide of lectin according to claim 19, which comprises the amino acid sequences shown in SEQ ID NOS: 3 to 9 by one for each.

21. A polypeptide of lectin, which comprises at least a part of the amino acid sequence shown in SEQ ID NO: 2, and may have substitution, deletion or insertion of one or more amino acid residues that does not substantially spoil its binding activity to a sugar chain.

22. A polypeptide of lectin which comprises at least a part of the amino acid sequence shown in SEQ ID NO: 2.

23. A polypeptide which comprises a portion of the polypeptide of lectin as defined in claim 21 or 22.

24. The polypeptide of lectin according to any one of claims 13 to 23, which has the amino acid sequence represented by the amino acid numbers 18-402 in SEQ ID NO: 2.

25. The polypeptide of lectin according to any one of claims 13 to 23, which has the amino acid sequence represented by the amino acid numbers 1-402 in SEQ ID NO: 2.

26. A fusion polypeptide which comprises the polypeptide as defined in any one of claims 13 to 25 and another polypeptide in its polypeptide chain.

27. The fusion polypeptide according to claim 26, wherein the other polypeptide is a peptide that can be biotinylated, glutathione-S-transferase, or gene 10 product of T7 phage.

28. An antibody which binds to the polypeptide as defined in any one of claims 13 to 27.

29. A DNA encoding a lectin having the following properties:
(1) binding to a sugar chain having an N-acetylglucosamine residue,
(2) having a molecular weight of about 40 kilodalton as estimated by sodium dodecyl sulfate-polyacrylamide gel electrophoresis,
(3) having the following amino acid composition:
| Residue | Mol % |
|---|---|
| Asx | 14.9 |
| Thr | 5.5 |
| Ser | 3.2 |
| Glx | 4.2 |
| Pro | 3.2 |
| Gly | 14.9 |
| Ala | 8.0 |
| Cys | 1.0 |
| Val | 10.4 |
| Met | 1.5 |
| Ile | 5.2 |
| Leu | 7.0 |
| Tyr | 2.5 |
| Phe | 5.5 |
| His | 1.7 |
| Lys | 3.7 |
| Trp | 2.2 |
| Arg | 5.2 |
(4) present in fruiting body of *Psathyrella velutina*.

30. A DNA which encodes the polypeptide as defined in any one of claims 13 to 20.

31. A DNA which encodes at least a portion of the polypeptide as defined in claim 21.

32. A DNA encoding a polypeptide of lectin, which has at least a part of a nucleotide sequence encoding the amino acid sequence shown in SEQ ID NO: 2.

33. A DNA which encodes the polypeptide as defined in claim 23.

34. A DNA which has a nucleotide sequence encoding the amino acid sequence represented by the amino acid numbers 18-402 in SEQ ID NO: 2.

35. A DNA which has a nucleotide sequence encoding the amino acid sequence represented by the amino acid numbers 1-402 in SEQ ID NO: 2.

36. A DNA which has at least a part or all of the nucleotide sequence represented by the nucleotide numbers 92-1246 in SEQ ID NO: 1.

37. A DNA which has at least a part or all of the nucleotide sequence represented by the nucleotide numbers 41-1246 in SEQ ID NO: 1.

38. A recombinant DNA which comprises the DNA as defined in any one of claims 29 to 37 which is ligated to a DNA encoding another polypeptide.

39. The recombinant DNA according to the claim 38, wherein the other polypeptide is a peptide that can be biotinylated, glutathione-S-transferase, or gene 10 product of T7 phage.

40. A recombinant vector which comprises the DNA as defined in any one of claims 29 to 39.

41. The recombinant vector according to claim 40, which is an expression vector.

42. The recombinant vector according to claim 41, wherein the expression vector comprises a nucleotide sequence encoding a peptide that can be biotinylated, glutathione-S-transferase, or gene 10 product of T7 phage.

43. A transformant cell which is obtainable by introducing the recombinant vector as defined in any one of claims 40 to 42 into a host cell.

44. The transformant cell according to claim 43, wherein the host cell is of *Escherichia coli* or yeast.

45. The method according to any one of claims 1 to 4, wherein the lectin is a lectin comprising the polypeptide as defined in any one of claims 13 to 25.

46. The kit for diagnosing rheumatism according to claim 12, wherein the lectin is a lectin comprising the polypeptide as defined in any one of claims 13 to 25.


SEQUENCE LISTING
(1) GENERAL INFORMATION:
(i) APPLICANT: Seikagaku Corporation
(ii) TITLE OF INVENTION: Method for Assaying Agalacto-IgG, Assay Kit,
Polypeptide of Lectin, and DNA Encoding the Same
(iii) NUMBER OF SEQUENCES: 9
(iv) CORRESPONDENCE ADDRESS:
(A) ADDRESSEE: Seikagaku Corporation
(B) STREET: 1-5, Nihonbashi-honcho 2-chome
(C) CITY: Chuo-ku
(D) STATE: Tokyo
(E) COUNTRY: JAPAN
(F) ZIP: 103
(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn
(vi) CURRENT APPLICATION DATA:
(A) APPLICATION NUMBER:
(B) FILING DATE: 15.10.97
(C) CLASSIFICATION:
(D) REFERENCE: 97-050P618
(vii) PRIOR APPLICATION DATA:
(A) APPLICATION NUMBER: JP 1996272731
(B) FILING DATE: 15.10.96
(vii) PRIOR APPLICATION DATA:
(A) APPLICATION NUMBER: JP 1996272732
(B) FILING DATE: 15.10.96
(vii) PRIOR APPLICATION DATA:
(A) APPLICATION NUMBER: JP 1997197628
(B) FILING DATE: 23.07.97

(2) INFORMATION FOR SEQ ID NO:1:
(i) SEQUENCE CHARACTERISTICS:
(A) LENGTH: 1256
(B) TYPE: nucleic acid
(C) STRANDEDNESS: double
(D) TOPOLOGY: linear
(ii) MOLECULAR TYPE: cDNA

(vi) ORIGINAL SOURCE:
(A) ORGANISM: Psathyrella velutina
(B) TISSUE TYPE: fruiting body
(ix) FEATURE:
(A) NAME/KEY: CDS
(B) LOCATION: 41..1246
(xi) SEQUENCE DESCRIPTION: SEQ ID NO:1:
ACCTCCTACG ATCTTCACCG CCTAAGAGAT ACAATACCCA ATG TCG ATC CCA GTC 55
Met Ser Ile Pro Val
1               5
ATC AGT CAA GCT TCG CCC GTC CCC ACC CGC ATT CCA GGT GTC GCA GAC 103
Ile Ser Gln Ala Ser Pro Val Pro Thr Arg Ile Pro Gly Val Ala Asp
                10                  15                  20
CTG GTC GGG TTC GGA ACT GGA GGA GTC TAC ATA ATC CGT AAC TCC CTC 151
Leu Val Gly Phe Gly Thr Gly Gly Val Tyr Ile Ile Arg Asn Ser Leu
            25                  30                  35
CTC ATC CAG GTC GTC AAA GTC ATC AAC AAC TTC GGC TAC GAC GCC GGA 199
Leu Ile Gln Val Val Lys Val Ile Asn Asn Phe Gly Tyr Asp Ala Gly
        40                  45                  50
GGA TGG CGC GTC GAA AAG CAC GTC CGC CTC CTC GCA GAC ACC ACA GGC 247
Gly Trp Arg Val Glu Lys His Val Arg Leu Leu Ala Asp Thr Thr Gly
    55                  60                  65
GAC AAC CAA TCT GAT GTG GTC GGC TTC GGC GAG AAC GGC GTC TGG ATC 295
Asp Asn Gln Ser Asp Val Val Gly Phe Gly Glu Asn Gly Val Trp Ile
70                  75                  80                  85
TCG ACC AAC AAC GGC AAC AAC ACG TTC ACC GAC CCC CCC AAG ATG GTG 343
Ser Thr Asn Asn Gly Asn Asn Thr Phe Thr Asp Pro Pro Lys Met Val
                90                  95                  100
ATT GCC AAC TTC GCA TAC AAC GCG GGC GGC TGG CGT GTC GAA AAG CAC 391
Ile Ala Asn Phe Ala Tyr Asn Ala Gly Gly Trp Arg Val Glu Lys His
            105                 110                 115
ATC CGT TTC ATG GCG GAC CTG CGC AAG ACC GGC CGT GCT GAC ATC GTC 439
Ile Arg Phe Met Ala Asp Leu Arg Lys Thr Gly Arg Ala Asp Ile Val
        120                 125                 130
GGG TTC GGA GAG GCC GGC ATC CTC GTC TCG CTC AAC AAC GGC GGC AGC 487
Gly Phe Gly Glu Ala Gly Ile Leu Val Ser Leu Asn Asn Gly Gly Ser
    135                 140                 145
CAG TTC GCG CCC GCC CAG CTC GCC TTG AAC AAC TTT GGG TAC GCC CAA 535
Gln Phe Ala Pro Ala Gln Leu Ala Leu Asn Asn Phe Gly Tyr Ala Gln
150                 155                 160                 165
GGA TGG AGG TTG GAC CGC CAC CTC CGT TTC CTC GGT GAC ATC ACC GGC 583

Gly Trp Arg Leu Asp Arg His Leu Arg Phe Leu Gly Asp Ile Thr Gly
                170                 175                 180
GAC GGC CTC CTC GAC GTT GTC GGT TTC GGC GAG AAC CAC GTC TAC GCC 631
Asp Gly Leu Leu Asp Val Val Gly Phe Gly Glu Asn His Val Tyr Ala
            185                 190                 195
GCA CGC AAC AAC GGC AAC GGC ACC TTC CAG CCT GCC CAG GCC GTC GTC 679
Ala Arg Asn Asn Gly Asn Gly Thr Phe Gln Pro Ala Gln Ala Val Val
        200                 205                 210
AAC AAC TTC TGC GTC GGC GCA GGA GGA TGG ACT ATC GCC TCC CAC CCT 727
Asn Asn Phe Cys Val Gly Ala Gly Gly Trp Thr Ile Ala Ser His Pro
    215                 220                 225
CGT GTC ATC GCC GAC CTC ACT GGA GAC AAG AGG GCC GAC ATC CTT GGC 775
Arg Val Ile Ala Asp Leu Thr Gly Asp Lys Arg Ala Asp Ile Leu Gly
230                 235                 240                 245
TTC GGC GGA GCA GGA GTG TAC ACC TCC CTC AAC AAC GGC AAC GGC ACT 823
Phe Gly Gly Ala Gly Val Tyr Thr Ser Leu Asn Asn Gly Asn Gly Thr
                250                 255                 260
TTC GGC GCC GTC AAC CTC GTC TTG AAG GAC TTT GGC ACC GCC AGC GGA 871
Phe Gly Ala Val Asn Leu Val Leu Lys Asp Phe Gly Thr Ala Ser Gly
            265                 270                 275
TGG CGT GTC GAG AAA CAC GTC CGC TGC GTC GCC CCC CTC ACC AAC AAG 919
Trp Arg Val Glu Lys His Val Arg Cys Val Ala Pro Leu Thr Asn Lys
        280                 285                 290
AAG GTC GGA GAC ATC ATC GGG TTC GGC GAC GCG GGC GTG TAC GTC GCG 967
Lys Val Gly Asp Ile Ile Gly Phe Gly Asp Ala Gly Val Tyr Val Ala
    295                 300                 305
CTC AAC AAT GGC AAC GGA ACG TTC GGC CCC GTC AAG CGC GTC ATC GAT 1015
Leu Asn Asn Gly Asn Gly Thr Phe Gly Pro Val Lys Arg Val Ile Asp
310                 315                 320                 325
AAC TTT GGG TAC AAC CAG GGA TGG CGA GTG GAC AAG CAC CCG AGG TTC 1063
Asn Phe Gly Tyr Asn Gln Gly Trp Arg Val Asp Lys His Pro Arg Phe
                330                 335                 340
GTC GTC GAC TTG ACC GGC GAC GGC TGC GCC GAT ATC GTC GGA TTT GGA 1111
Val Val Asp Leu Thr Gly Asp Gly Cys Ala Asp Ile Val Gly Phe Gly
            345                 350                 355
GAG AAC TCG GTT TGG GCG TGC ATG AAC AAG GGC GAC GGA ACC TTT GGA 1159
Glu Asn Ser Val Trp Ala Cys Met Asn Lys Gly Asp Gly Thr Phe Gly
        360                 365                 370
CCG ATG ATG AAG CTG ATT GAC GAC TTG ACG GTC TCC AAG GGC TGG ACC 1207
Pro Met Met Lys Leu Ile Asp Asp Leu Thr Val Ser Lys Gly Trp Thr
    375                 380                 385

CTC CAG AGG ACC GTC CGA TAC GCC GCG AAC CTC TAC CTC TGAACGCGAC 1256
Leu Gln Arg Thr Val Arg Tyr Ala Ala Asn Leu Tyr Leu
390                 395                 400

(2) INFORMATION FOR SEQ ID NO:2:
(i) SEQUENCE CHARACTERISTICS:
(A) LENGTH: 402
(B) TYPE: amino acid
(D) TOPOLOGY: linear
(ii) MOLECULAR TYPE: protein
(xi) SEQUENCE DESCRIPTION: SEQ ID NO:2:
Met Ser Ile Pro Val Ile Ser Gln Ala Ser Pro Val Pro Thr Arg Ile
1               5                   10                  15
Pro Gly Val Ala Asp Leu Val Gly Phe Gly Thr Gly Gly Val Tyr Ile
            20                  25                  30
Ile Arg Asn Ser Leu Leu Ile Gln Val Val Lys Val Ile Asn Asn Phe
        35                  40                  45
Gly Tyr Asp Ala Gly Gly Trp Arg Val Glu Lys His Val Arg Leu Leu
    50                  55                  60
Ala Asp Thr Thr Gly Asp Asn Gln Ser Asp Val Val Gly Phe Gly Glu
65                  70                  75                  80
Asn Gly Val Trp Ile Ser Thr Asn Asn Gly Asn Asn Thr Phe Thr Asp
                85                  90                  95
Pro Pro Lys Met Val Ile Ala Asn Phe Ala Tyr Asn Ala Gly Gly Trp
            100                 105                 110
Arg Val Glu Lys His Ile Arg Phe Met Ala Asp Leu Arg Lys Thr Gly
        115                 120                 125
Arg Ala Asp Ile Val Gly Phe Gly Glu Ala Gly Ile Leu Val Ser Leu
    130                 135                 140
Asn Asn Gly Gly Ser Gln Phe Ala Pro Ala Gln Leu Ala Leu Asn Asn
145                 150                 155                 160
Phe Gly Tyr Ala Gln Gly Trp Arg Leu Asp Arg His Leu Arg Phe Leu
                165                 170                 175
Gly Asp Ile Thr Gly Asp Gly Leu Leu Asp Val Val Gly Phe Gly Glu
            180                 185                 190
Asn His Val Tyr Ala Ala Arg Asn Asn Gly Asn Gly Thr Phe Gln Pro
        195                 200                 205
Ala Gln Ala Val Val Asn Asn Phe Cys Val Gly Ala Gly Gly Trp Thr
    210                 215                 220
Ile Ala Ser His Pro Arg Val Ile Ala Asp Leu Thr Gly Asp Lys Arg
225                 230                 235                 240

Ala Asp Ile Leu Gly Phe Gly Gly Ala Gly Val Tyr Thr Ser Leu Asn
                245                 250                 255
Asn Gly Asn Gly Thr Phe Gly Ala Val Asn Leu Val Leu Lys Asp Phe
            260                 265                 270
Gly Thr Ala Ser Gly Trp Arg Val Glu Lys His Val Arg Cys Val Ala
        275                 280                 285
Pro Leu Thr Asn Lys Lys Val Gly Asp Ile Ile Gly Phe Gly Asp Ala
    290                 295                 300
Gly Val Tyr Val Ala Leu Asn Asn Gly Asn Gly Thr Phe Gly Pro Val
305                 310                 315                 320
Lys Arg Val Ile Asp Asn Phe Gly Tyr Asn Gln Gly Trp Arg Val Asp
                325                 330                 335
Lys His Pro Arg Phe Val Val Asp Leu Thr Gly Asp Gly Cys Ala Asp
            340                 345                 350
Ile Val Gly Phe Gly Glu Asn Ser Val Trp Ala Cys Met Asn Lys Gly
        355                 360                 365
Asp Gly Thr Phe Gly Pro Met Met Lys Leu Ile Asp Asp Leu Thr Val
    370                 375                 380
Ser Lys Gly Trp Thr Leu Gln Arg Thr Val Arg Tyr Ala Ala Asn Leu
385                 390                 395                 400
Tyr Leu

(2) INFORMATION FOR SEQ ID NO:3:
     (i) SEQUENCE CHARACTERISTICS:
          (A) LENGTH: 53
          (B) TYPE: amino acid
          (D) TOPOLOGY: linear
    (ii) MOLECULAR TYPE: peptide
    (xi) SEQUENCE DESCRIPTION: SEQ ID NO:3:
Gly Val Ala Asp Leu Val Gly Phe Gly Thr Gly Gly Val Tyr Ile Ile
  1               5                  10                  15
Arg Asn Ser Leu Leu Ile Gln Val Val Lys Val Ile Asn Asn Phe Gly
             20                  25                  30
Tyr Asp Ala Gly Gly Trp Arg Val Glu Lys His Val Arg Leu Leu Ala
         35                  40                  45
Asp Thr Thr Gly Asp
     50

(2) INFORMATION FOR SEQ ID NO:4:
     (i) SEQUENCE CHARACTERISTICS:

(A) LENGTH: 57
(B) TYPE: amino acid
(D) TOPOLOGY: linear
(ii) MOLECULAR TYPE: peptide
(xi) SEQUENCE DESCRIPTION: SEQ ID NO:4:
Asn Gln Ser Asp Val Val Gly Phe Gly Glu Asn Gly Val Trp Ile Ser
1               5                   10                  15
Thr Asn Asn Gly Asn Asn Thr Phe Thr Asp Pro Pro Lys Met Val Ile
            20                  25                  30
Ala Asn Phe Ala Tyr Asn Ala Gly Gly Trp Arg Val Glu Lys His Ile
        35                  40                  45
Arg Phe Met Ala Asp Leu Arg Lys Thr
    50                  55

(2) INFORMATION FOR SEQ ID NO:5:
(i) SEQUENCE CHARACTERISTICS:
(A) LENGTH: 55
(B) TYPE: amino acid
(D) TOPOLOGY: linear
(ii) MOLECULAR TYPE: peptide
(xi) SEQUENCE DESCRIPTION: SEQ ID NO:5:
Gly Arg Ala Asp Ile Val Gly Phe Gly Glu Ala Gly Ile Leu Val Ser
1               5                   10                  15
Leu Asn Asn Gly Gly Ser Gln Phe Ala Pro Ala Gln Leu Ala Leu Asn
            20                  25                  30
Asn Phe Gly Tyr Ala Gln Gly Trp Arg Leu Asp Arg His Leu Arg Phe
        35                  40                  45
Leu Gly Asp Ile Thr Gly Asp
    50                  55

(2) INFORMATION FOR SEQ ID NO:6:
(i) SEQUENCE CHARACTERISTICS:
(A) LENGTH: 56
(B) TYPE: amino acid
(D) TOPOLOGY: linear
(ii) MOLECULAR TYPE: peptide
(xi) SEQUENCE DESCRIPTION: SEQ ID NO:6:
Gly Leu Leu Asp Val Val Gly Phe Gly Glu Asn His Val Tyr Ala Ala
1               5                   10                  15
Arg Asn Asn Gly Asn Gly Thr Phe Gln Pro Ala Gln Ala Val Val Asn
            20                  25                  30

Asn Phe Cys Val Gly Ala Gly Gly Trp Thr Ile Ala Ser His Pro Arg
        35                  40                  45
Val Ile Ala Asp Leu Thr Gly Asp
    50                  55

(2) INFORMATION FOR SEQ ID NO:7:
     (i) SEQUENCE CHARACTERISTICS:
          (A) LENGTH: 55
          (B) TYPE: amino acid
          (D) TOPOLOGY: linear
    (ii) MOLECULAR TYPE: peptide
    (xi) SEQUENCE DESCRIPTION: SEQ ID NO:7:
Lys Arg Ala Asp Ile Leu Gly Phe Gly Gly Ala Gly Val Tyr Thr Ser
 1               5                  10                  15
Leu Asn Asn Gly Asn Gly Thr Phe Gly Ala Val Asn Leu Val Leu Lys
            20                  25                  30
Asp Phe Gly Thr Ala Ser Gly Trp Arg Val Glu Lys His Val Arg Cys
        35                  40                  45
Val Ala Pro Leu Thr Asn Lys
    50                  55

(2) INFORMATION FOR SEQ ID NO:8:
     (i) SEQUENCE CHARACTERISTICS:
          (A) LENGTH: 55
          (B) TYPE: amino acid
          (D) TOPOLOGY: linear
    (ii) MOLECULAR TYPE: peptide
    (xi) SEQUENCE DESCRIPTION: SEQ ID NO:8:
Lys Val Gly Asp Ile Ile Gly Phe Gly Asp Ala Gly Val Tyr Val Ala
 1               5                  10                  15
Leu Asn Asn Gly Asn Gly Thr Phe Gly Pro Val Lys Arg Val Ile Asp
            20                  25                  30
Asn Phe Gly Tyr Asn Gln Gly Trp Arg Val Asp Lys His Pro Arg Phe
        35                  40                  45
Val Val Asp Leu Thr Gly Asp
    50                  55

(2) INFORMATION FOR SEQ ID NO:9:
     (i) SEQUENCE CHARACTERISTICS:
          (A) LENGTH: 54
          (B) TYPE: amino acid

(D) TOPOLOGY: linear
(ii) MOLECULAR TYPE: peptide
(xi) SEQUENCE DESCRIPTION: SEQ ID NO:9:
Gly Cys Ala Asp Ile Val Gly Phe Gly Glu Asn Ser Val Trp Ala Cys
1               5                   10                  15
Met Asn Lys Gly Asp Gly Thr Phe Gly Pro Met Met Lys Leu Ile Asp
            20                  25                  30
Asp Leu Thr Val Ser Lys Gly Trp Thr Leu Gln Arg Thr Val Arg Tyr
        35                  40                  45
Ala Ala Asn Leu Tyr Leu
    50